# EUROPÄISCHE PATENTSCHRIFT

(11) **EP 2 488 510 B1**
(45) Veröffentlichungstag und Bekanntmachung des Hinweises auf die Patenterteilung: **29.03.2017**
(21) Anmeldenummer: 10762928.9
(22) Anmeldetag: 08.10.2010
(51) Int. Cl.: C07D 401/04, C07D 401/14, C07D 407/14, C07D 413/14, A01N 43/56

(54) **Amide und Thioamide als Schädlingsbekämpfungsmittel**
Amides and thioamides as pest control agents
Amides et thioamides en tant qu'agents pesticides

(30) Priorität: 12.10.2009 EP 09172736; 13.10.2009 US 251042 P
(43) Veröffentlichungstag der Anmeldung: 22.08.2012
(73) Patentinhaber: Bayer Intellectual Property GmbH, 40789 Monheim am Rhein (DE)
(72) Erfinder: BRETSCHNEIDER, Thomas, 53797 Lohmar (DE); VOERSTE, Arnd, 50674 Köln (DE); FÜßLEIN, Martin, 40225 Düsseldorf (DE); KÖHLER, Adeline, 42105 Wuppertal (DE); GÖRGENS, Ulrich, 40882 Ratingen (DE)
(74) Vertreter: BIP Patents
(86) Internationale Anmeldenummer: PCT/EP2010/065104
(87) Internationale Veröffentlichungsnummer: WO 2011/045240

(56) Entgegenhaltungen:
- EP-A- 1 938 686
- DE-A1- 2 221 647
- DE-A1-102006 032 168

## Beschreibung

Die vorliegende Anmeldung betrifft neue Amide und Thioamide, Verfahren zu Ihrer Herstellung und ihre Verwendung zur Bekämpfung von tierischen Schädlingen, zu denen Arthropoden und insbesondere Insekten zählen.

Bestimmte Amide sind bereits als insektizid wirksame Verbindungen bekannt geworden (vgl. DE 2221647).

Pyridinyl-Pyrazol-Essigsäuren werden ebenfalls als Pflanzenwachstumsregulatoren eingesetzt (vgl. EP 1 938 686).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I), 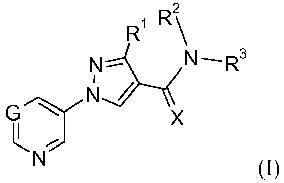 worin
- G: für N, CH, C-Halogen, C-Nitro, C-Cyano, C-Alkyl, C-Haloalkyl, C-Cycloalkyl, C-Alkoxy, C-Haloalkoxy steht,
- R¹: für Wasserstoff, Alkyl, Haloalkyl, Cycloalkyl, Halogen, Cyano, Alkoxy, Haloalkoxy, Amino, Alkylamino, Dialkylamino oder Thioalkyl steht,
- X: für Sauerstoff oder Schwefel steht,
- R²: für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht,
und
- R³: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, gegebenenfalls am Stickstoff durch Alkyl substituiertes Alkoxycarbonylamino, Alkinyloxy, Alkinyloxycarbonyl, gegebenenfalls substituiertes Cycloalkyl, gegebenenfalls substituiertes Cycloalkylcarbonyl, gegebenenfalls substituiertes Cycloalkylalkyl, gegebenenfalls substituiertes Heterocyclyl, gegebenenfalls substituiertes Heterocyclylalkyl, gegebenenfalls substituiertes Arylalkyl, gegebenenfalls durch Halogen substituiertes Aryloxyalkyl, gegebenenfalls substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloyalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Heterocyclus bilden,
oder
- R² und R³: gemeinsam mit dem Stickstoffatom, an das sie gebunden sind, einen gegebenenfalls substituierten und gegebenenfalls weitere Heteroatome enthaltenden Ring bilden,
sowie Salze und N-Oxide der Verbindungen der Formel (I).

Insbesondere gelöst wird die oben genannte Aufgabe, sowie weitere nicht explizit genannte Aufgaben, die aus den hierin diskutierten Zusammenhängen ableitbar oder erschließbar sind, durch neue Verbindungen der oben genannten Formel (I), worin
- G: für N, CH, C-Halogen, C-Nitro, C-Cyano, C-Alkyl, C-Haloalkyl, C-Cycloalkyl, C-Alkoxy, C-Haloalkoxy steht,

- R¹: für Wasserstoff, Alkyl, Haloalkyl, Cycloalkyl, Halogen, Cyano, Alkoxy, Haloalkoxy, Amino, Alkylamino, Dialkylamino oder Thioalkyl steht,
- X: für Sauerstoff oder Schwefel steht,
- R²: für einen Rest aus der Reihe Wasserstoff, Alkyl, Halogenalkyl, Alkoxy, Halogenalkoxy, Alkenyl, Alkoxyalkyl, gegebenenfalls durch Halogen substituiertes Alkylcarbonyl, gegebenenfalls durch Halogen substituiertes Alkoxycarbonyl und gegebenenfalls durch Halogen substituiertes Cycloalkylcarbonyl steht,
und
- R³: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, gegebenenfalls durch Halogen substituiertes Alkoxycarbonylalkyl, gegebenenfalls am Stickstoff durch Alkyl substituiertes Alkoxycarbonylamino, Alkinyloxy, Alkinyloxycarbonyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes und gegebenenfalls benzokondensiertes Heterocyclylalkyl, gegebenenfalls durch Halogen, Cyano, Alkyl, Halogenalkyl, Alkoxy oder Halogenalkoxy substituiertes Arylalkyl, gegebenenfalls durch Halogen substituiertes Aryloxyalkyl, gegebenenfalls durch Halogen, Cyano (auch im Alkylteil), Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfmyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl und NR⁴R⁵ steht, worin R⁴ und R⁵ unabhängig voneinander für einen Rest aus der Reihe Wasserstoff, Alkyl, Halogenalkyl, Cycloyalkyl, Alkoxy, Alkylcarbonyl, Alkoxycarbonyl, Hetaryl und Heterocyclyl stehen oder R⁴ und R⁵ gemeinsam mit dem Stickstoffatom, an das sie gebunden sind, einen gegebenenfalls substituierten Hetrocyclus bilden
oder
- R² und R³: gemeinsam mit dem Stickstoffatom, an das sie gebunden sind, einen gegebenenfalls substituierten und gegebenenfalls weitere Heteroatome enthaltenden Ring bilden,
sowie Salze und N-Oxide der Verbindungen der Formel (I).

Weiter wurde gefunden, dass man die neuen Verbindungen der Formel (I) erhält, wenn man Verbindungen der Formel (II) 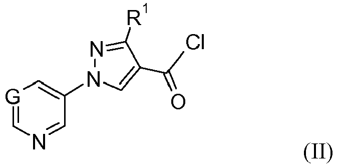 in welcher
G und R¹ die weiter oben genannten Bedeutungen haben
mit Verbindungen der Formel (III) 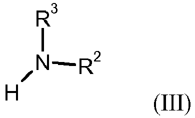 in welcher
R²und R³ die weiter oben genannten Bedeutungen haben, gegebenenfalls in Gegenwart eines geeigneten Verdünnungsmittels und gegebenenfalls in Gegenwart eine Base umsetzt.

Die nach diesen Verfahren erhältlichen Verbindungen der Formel (I), in denen X für Sauerstoff steht, lassen sich durch Umsetzung mit einem Schwefelungsreagenz in Verbindungen der Formel (I) überführen, in denen X für Schwefel steht.

Schließlich wurde gefunden, das die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorratsund Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen, wie sie für andere Amide beispielsweise in DE 2221647 beschrieben sind.

Bevorzugte Substituenten bzw. Bereiche der in der oben erwähnten Verbindungen der Formel (I), aufgeführten Reste werden im Folgenden erläutert. Dabei wird der Rest Pyrimidyl auch als Pyrimidinyl und der Rest Furyl auch als Furanyl bezeichnet.
- G: steht für N, CH, C-Halogen, C-Nitro, C-Cyano, C-C₁-C₆-Alkyl, C-C₁-C₆-Haloalkyl, C-C₃-C₆-Cycloalkyl, C-C₁-C₆-Alkoxy, C-C₁-C₆-Haloalkoxy, insbesondere für N, CH, C-Halogen, C-Cyano, C-Trifluoromethyl,
- R¹: steht für Wasserstoff, C₁-C₆-Alkyl, C₁-C₆-Haloalkyl, C₃-C₆-Cycloalkyl, Halogen, Cyano, C₁-C₆-Alkoxy, C₁-C₆-Haloalkoxy, Amino, C₁-C₆-Alkylamino, Di-(C₁-C₆)-alkylamino oder C₁-C₆-Thioalkyl, insbesondere für Wasserstoff und Methyl,
- X: steht für Sauerstoff oder Schwefel.
- R²: steht für einen Rest aus der Reihe Wasserstoff, C₁-C₆-Alkyl, C₁-C₆-Halogenalkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl und gegebenenfalls durch Halogen substituiertes C₃-C₆-Cycloalkylcarbonyl.
- R³: steht für einen Rest aus der Reihe Wasserstoff, C₁-C₆-Alkyl, C₁-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylthio-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfmyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, gegebenenfalls am Stickstoff durch C₁-C₆-Alkyl substituiertes C₁-C₆-Alkoxycarbonylamino, C₂-C₄-Alkinyloxy, C₂-C₄-Alkinyloxycarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfmyl, C₁-C₆-Alkylsulfonyl, C₁-C₆-Haloalkylsulfmyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes und gegebenenfalls benzokondensiertes Heterocyclyl-C₁-C₆-alkyl, gegebenenfalls durch Halogen, Cyano, C₁-C₆-Alkyl, C₁-C₆-Halogenalkyl, C₁-C₆-Alkoxy oder C₁-C₆-Halogenalkoxy substituiertes Aryl-C₁-C₆-alkyl, gegebenenfalls im Arylteil durch Halogen substituiertes Aryloxy-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Aklsulfinyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl, und NR⁴R⁵, worin R⁴ und R⁵ unabhängig voneinander für einen Rest aus der Reihe Wasserstoff, C₁-C₆-Alkyl, C₁-C₆-Halogenalkyl, C₃-C₆-Cycloyalkyl, C₁-C₆-Alkoxy, C₁-C₆-Alkylcarbonyl und C₁-C₆-Alkoxycarbonyl stehen,
oder
- R² und R³: bilden gemeinsam mit dem Stickstoffatom, an das sie gebunden sind, einen gegebenenfalls durch C₁-C₆-Alkyl oder C₁-C₆-Alkoxy substituierten und gegebenenfalls ein oder zwei weitere Heteroatome aus der Reihe Sauerstoff, Stickstoff und Schwefel enthaltenden 3 bis 7 gliedrigen Ring.

Besonders bevorzugte Substituenten bzw. Bereiche der in den erfindungsgemäßen Verbindungen der Formel (I) aufgeführten Reste werden im Folgenden erläutert.
- G: steht für N, CH, C-Halogen, C-Nitro, C-Cyano, C-C₁-C₄-Alkyl, C-C₁-C₄-Haloalkyl, C-C₃-C₆-Cycloalkyl, C-C₁-C₄-Alkoxy, C-C₁-C₄-Haloalkoxy, insbesondere für N, CH, C-Halogen, C-Cyano oder C-Trifluoromethyl.
- R¹: steht für Wasserstoff, C₁-C₄-Alkyl, C₁-C₄-Haloalkyl, C₃-C₆-Cycloalkyl, Halogen, Cyano, C₁-C₄-Alkoxy, C₁-C₄-Haloalkoxy, Amino, C₁-C₄-Alkylamino, Di-(C₁-C₄)-alkylamino oder C₁-C₄-Thioalkyl, insbesondere für Wasserstoff oder Methyl.
- X: steht für Sauerstoff oder Schwefel.
- R²: steht für einen Rest aus der Reihe Wasserstoff, C₁-C₄-Alkyl, C₁-C₄-Halogenalkyl, C₁-C₄-Alkoxy, C₂-C₃-Alkenyl, C₁-C₂-Alkoxy-C₁-C₂-alkyl, C₁-C₂-Alkylcarbonyl, C₁-C₂-Alkoxycarbonyl und jeweils gegebenenfalls durch Halogen substituiertes Cyclopropylcarbonyl, Cyclopentylcarbonyl oder Cyclohexylcarbonyl.
- R³: steht für einen Rest aus der Reihe Wasserstoff, C₁-C₄-Alkyl, C₁-C₅-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylthio-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, gegebenenfalls am Stickstoff durch C₁-C₄-Alkyl substituiertes C₁-C₄-Alkoxycarbonylamino, C₂-C₄-Alkinyloxy, C₂-C₄-Alkinyloxycarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes und gegebenenfalls benzokondensiertes Heterocyclyl-C₁-C₄-alkyl, gegebenenfalls durch Halogen, Cyano, C₁-C₄-Alkyl, C₁-C₄-Halogenalkyl, C₁-C₄-Alkoxy oder C₁-C₄-Halogenalkoxy substituiertes Aryl-C₁-C₄-alkyl, gegebenenfalls im Arylteil durch Halogen substituiertes Aryloxy-C₁-C₄-alkyl und gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl
oder
- R² und R³: bilden gemeinsam mit dem Stickstoffatom, an das sie gebunden sind, einen gegebenenfalls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring, R² und R³ stehen gemeinsam beispielsweise für CH₂CH₂CH₂ oder für CH₂CH₂CH₂O.

Ganz besonders bevorzugte Substituenten bzw. Bereiche der in den erfindungsgemäßen Verbindungen der Formel (I) aufgeführten Reste werden im Folgenden erläutert.
- G: steht für N, CH, C-Halogen, C-Cyano, C-CH₃, C-CF₃, C-Cyclopropyl, C-OCH₃, C-OCF₃, insbesondere für N, CH, C-Halogen,
- R¹: steht für Wasserstoff, Methyl, Trifluoromethyl, Cyclopropyl, Halogen, Cyano, Methoxy, Trifluoromethoxy, Amino, Methylamino, Dimethylamino, insbesondere für Wasserstoff und Methyl.
- X: steht für Sauerstoff oder Schwefel.
- R²: steht für einen Rest aus der Reihe Wasserstoff, Methyl, Ethyl, CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, Methoxy, Ethoxy, Vinyl, CH₂OCH₃, CH₂OCH₂CH₃, Methylcarbonyl, Ethylcarbonyl, Methoxycarbonyl, Ethoxycarbonyl, Cyclopropylcarbonyl und Fluorcyclpropylcarbonyl.
- R³: steht für einen Rest aus der Reihe Wasserstoff, Methyl, Ethyl, CH₂CF₃, CH₂CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, CH(CN)CH(CH₃)₂, CH₂CN, CH₂CH₂CN, Vinyl, C(CH₃)₂CCH, CH₂CCCH₃, Methoxy, Ethoxy, CH₂CH(OCH₃)₂, CH₂CH(CH₃)(OCH₃), CH₂C(CH₃)₂(OCH₃), CH(CH₃)CH(OCH₃)₂, CH₂C(CH₃)(OCH₃)₂, C(CH₃)₂CH₂SCH₃, CH₂CH₂SCH₃, CHCH₃CH₂SCH₃, gegebenenfalls durch Halogen substituiertes C₁-C₄-Alkylcarbonyl-C₁-C₄-alkyl, CH₃SO₂CH₂C(CH₃)₂, CH₃SO₂CH₂CHCH₃, Propargyloxy, Cyclopropyl, Cyanocyclopropyl, Fluorcyclopropyl, Trifluormethylcyclopropyl, Trifluormethylcyclohexyl, Methoxycarbonylcyclopropyl, Fluorcyclopropylcarbonyl, Cyclopropylmethyl, 1-Cyclopropylethyl, Cyclohexylmethyl, 1-Cyano-1-cyclopropyl-eth-1-yl, 1,3-Dioxolan-2-ylmethyl, 4-Methyl-1,3-Dioxolan-2-ylmethyl, Tetra-hydrofurylmethyl, Tetrahydropyranylmethyl, 2,2-Dimethyl-1,3-dioxolan-5-yl-methyl, 2-Methyltetrahydrofur-2-yl-methyl, α-Methyl-3,5-dimethyltriazol-1-yl-ethyl, 1,5-Dimethyl-1,3-oxazol-4-yl-methyl, gegebenenfalls durch Halogen, Cyano, Methyl, Ethyl, Methoxy oder Ethoxy substituiertes Benzyl, für Pyrimidylmethyl, Pyridylmethyl, α-Methyl-pyrimidylmethyl, Oxadiazolylmethyl, Oxazolylmethyl, 5-Methyl-pyrazin-2-yl, α-Methyl-pyrid-2-yl-methyl, 4-Brom-pyrimid-2-yl-methyl, Imidazolylmethyl, 2-Methylpyrimid-4-yl-methyl, 6-Chlor-pyridin-3-yl-methyl, 4,6-Dimethylpyrimid-2-yl-methyl, Thiazolylmethyl, Furanylmethyl, 1,5-Dimethylpyrazol-3-yl-methyl, 4-lod-pyrimid-2-yl-methyl, 3-Cyclopropyl-1,2,4-oxdiazol-5-yl-methyl, 2-Ethyl-pyrimid-6-yl-methyl, 6-Brom-pyrid-2-yl-methyl, (2,3-Dihydro-1-benzofur-2-yl)methyl, (1,4-Dioxan-2-yl)methyl, 1,3-Dioxolan-2-ylmethyl, Methoxycarbonylethyl (Methylalaninat), Methoxycarbonylamino, 4,6-Dimethoxypyrimid-2-ylmethyl, 2-(2,5-Dichlorphenoxy)ethyl und N-Methyl-N-Methoxycarbonyl-amino,
oder
- R² und R³: bilden gemeinsam mit dem Stickstoffatom, an das sie gebunden sind, einen gegebenenfalls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring, beispielsweise stehen R² und R³ gemeinsam für CH₂CH₂CH₂ oder CH₂CH₂CH₂O.

In den bevorzugten Definitionen ist, sofern nichts anderes angegeben ist,
Halogen (bzw. Halo) ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl (beispielsweise 1,3-Oxazol-4-yl), Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2,3-Dihydro-1-benzofur-2-yl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2 , 3-Dihydropyrazol-2-yl, 2,3-Dihydropyrazol-3-yl, 2,3-Dihydropyrazol-4-yl, 2, 3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 1,4-Dioxan-2-yl, 1,3-Dioxolan-2-yl, 1,3-Dioxolan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

In den besonders bevorzugten Definitionen ist, sofern nichts anderes angegeben ist,
Halogen (bzw.Halo) ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (auch als Teil einer größeren Einheit, wie beispielsweise Hetarylalkyl) ausgewählt aus der Reihe Pyrimidyl, Oxadiazolyl, Oxazolyl, Pyrazinyl, Imidazolyl, Thiazolyl und Furanyl,
Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2,3-Dihydro-1-benzofur-2-yl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2 , 3-Dihydropyrazol-2-yl, 2,3-Dihydropyrazol-3-yl, 2,3-Dihydropyrazol-4-yl, 2, 3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 1,4-Dioxan-2-yl, 1,3-Dioxolan-2-yl, 1,3-Dioxolan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

Durch Halogen substituierte Reste, z.B. Haloalkyl ("Halo" ist in Ausdrücken wie "Haloalkyl" gleichbedeutend mit "Halogen"), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Bevorzugt, besonders bevorzugt oder ganz besonders bevorzugt sind Verbindungen, welche jeweils die unter bevorzugt, besonders bevorzugt oder ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

In einer hervorgehobenen Gruppe von erfindungsgemäßen Verbindungen steht G für CH.

In einer weiteren hervorgehobenen Gruppe von erfindungsgemäßen Verbindungen steht R³ für Halogenalkyl.

In einer weiteren hervorgehobenen Gruppe von erfindungsgemäßen Verbindungen steht R³ für gegebenenfalls substituiertes Heterocyclylalkyl.

In einer weiteren hervorgehobenen Gruppe von erfindungsgemäßen Verbindungen steht R³ für gegebenenfalls substituiertes Hetarylalkyl.

In einer weiteren hervorgehobenen Gruppe von erfindungsgemäßen Verbindungen steht X für Sauerstoff.

In einer weiteren hervorgehobenen Gruppe von erfindungsgemäßen Verbindungen steht X für Schwefel.

Die Herstellung erfindungsgemäßer Verbindungen der Formel (I) und entsprechender Vorstufen werden in den folgenden Reaktionsschemata erläutert.

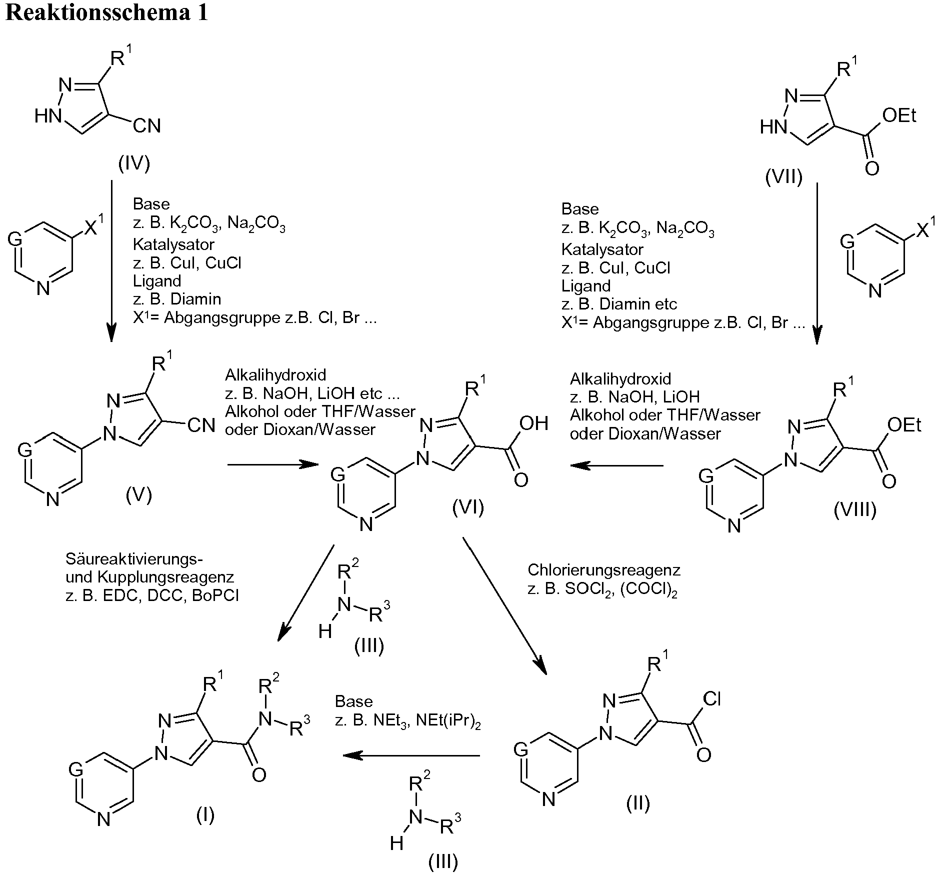

Die als Ausgangsstoffe benötigten Nitrile der Formel (V) sind bekannt bzw. nach bekannten Methoden erhältlich, beispielsweise wie für G gleich CH und R¹ gleich H in Journal of Heterocyclic Chemistry 1981, 18, 9-14 beschrieben. Die Nitrile der Formel (V) lassen sich durch N-Arylierung der Pyrazole (IV) mit 3-Brompyridin erhalten. Die als Ausgangsstoffe benötigten Ester der Formel (VIII) sind ebenfalls bekannt bzw. nach bekannten Methoden erhältlich, beispielsweise wie für G gleich CH und R¹ gleich H in Journal of Organic Chemistry 2004, 69, 5578-5587 beschrieben. Weitere Ausgangsstoffe der Formel (V) und (VIII), in welcher G und R¹ die oben genannte Bedeutung haben, lassen sich nach analogen Methoden herstellen.

Die Pyrazole der Formel (V) und (VIII) können unter Verwendung der in Reaktionsschema 1 angegebenen Standardmethoden, vgl. beispielsweise DE 2221647, zunächst in die Säuren der Formel (VI) und anschließend in die Säurechloride der Formel (II) überführt werden. Weitere Umsetzung mit den Aminen der Formel (III), in welcher R² und R³ die oben angegebenen Bedeutungen haben, in einem Verdünnungsmittel wie beispielsweise Dichlormethan oder Tetrahydrofuran und in Gegenwart einer Base wie beispielsweise Triethylamin oder Diisopropylethylamin, führt zu erfindungsgemäßen Verbindungen der Formel (I).

Die Verbindungen der Formel (I) lassen sich auch direkt aus den Säuren der Formel (VI) durch Umsetzung mit Aminen der Formel (III) in Gegenwart von Kupplungsagenzien wie beispielsweise EDC (1-Ethyl-3-[3-dimethylaminopropyl]carbodiimidehydrochlorid), DCC (Dicyclohexylcarbodiimid) oder BoPCl (Bis(2-oxo-3-oxazolidinyl)phosphisäurechlorid) herstellen.

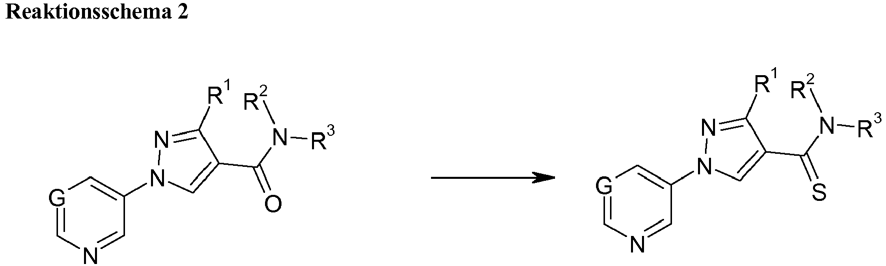

Die Umsetzung von Amiden zu Thioamiden kann mit P₄S₁₀ oder Lawesson Reagenz durchgeführt werden.

Für Umsetzungen mit Lawesson Reagenz siehe: Bull. Soc. Chem. Bel. 1978, 87, 223

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl als Saatgutanwendungen als auch in Vor-, Tank- oder Fertigmischungen verwendet werden.

Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Organochlorine, z.B. Chlordane und Endosulfan (alpha-); oder
   Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda), Cypermethrin (alpha-, beta-, theta-, zeta-), Cyphenothrin [(1*R*)-*trans*-Isomere], Deltamethrin, Dimefluthrin, Empenthrin [(*EZ*-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1R)- Isomere], Tralomethrin, Transfluthrin und ZXI 8901; oder
   DDT; oder Methoxychlor.
(4) Nikotinerge Acetylcholin-Rezeptor-Agonisten, wie beispielsweise
   Neonikotinoide, z.B. Acetamiprid, Clothianidin, Dinotefuran, Imidacloprid, Nitenpyram, Thiacloprid, Thiamethoxam; oder
   Nikotin.
(5) Allosterische Acetylcholin-Rezeptor-Modulatoren (Agonisten), wie beispielsweise
   Spinosyne, z.B. Spinetoram und Spinosad.
(6) Chlorid-Kanal-Aktivatoren, wie beispielsweise
   Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen 4-{[(6-Brompyrid-3-yl)methyl](2-fluorethyl)amino}furan-2(5H)-on **(bekannt aus** WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(SH)-**on** (**bekannt aus** WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 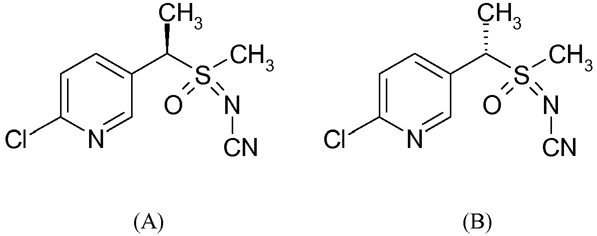 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor (bekannt aus WO 2007/149134), 11-(4-Chlor-2,6-dimethylphenyl)-12-hydroxy-1,4-dioxa-9-azadispiro[4.2.4.2]tetra-dec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911), 1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und N-[1-(2,3-Dimethylphenyl)-2-(3,5-dimethylphenyl)ethyl]-4,5-dihydro-1,3-thiazol-2-amin (bekannt aus WO2008/104503).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und Cry-IF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufinilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Beispiel A

### Stufe 1: 1-(Pyridin-3-yl)-1H-pyrazol-4-carbonitril

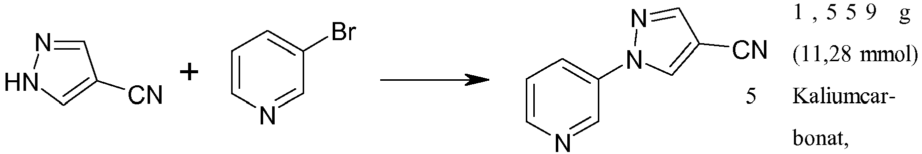

0,051 g (0,269mmol) Kupferiodid, 0,153 g (1,074mmol) Trans-N,N'-Dimethyl-1,2-cyclohexandiamin und 0,500 g (5,371 mmol) 4-Cyanopyrazol wurden unter Argon in 2,07 ml (21,49 mmol) 3-Brompyridin gelöst. Die Mischung wurde 24 h auf 110 °C erhitzt und anschließend auf Raumtemperatur abgekühlt. Dichloromethan wurde zugegeben und der Feststoff abfiltriert. Das Filtrat wurde eingeengt und der Rückstand mit Diethylether verrührt. Der gebildete Niederschlag wurde abfiltriert und am Rotationsverdampfer getrocknet.
Ausbeute: 0,710 mg (78% d. Th.), logP¹⁾ (HCOOH) 0,98
¹H-NMR((CD₃)₂SO): 7,59 (m, 1H), 8,24 (m, 1H), 8,36 (s, 1H), 8,63 (m, 1H), 9,09 (m, 1H), 9,33 (s, 1H)

### Stufe 2: 1-(Pyridin-3-yl)-1H-pyrazol-4-carbonsäure

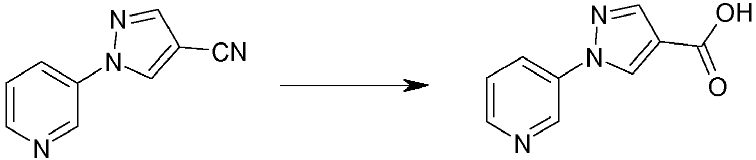

8,360 g (49,13 mmol) 1-(Pyridin-3-yl)-1H-pyrazol-4-carbonitril wurden in ca. 200 ml Dioxan gelöst und mit ca. 15 ml Wasser und 10,48 g einer 45 %igen wässrigen Natriumhydroxidlösung versetzt. Die Mischung wurde 12 h unter Rückfluß erhitzt. Da die Umsetzung nicht vollständig war, wurden zusätzlich 10,48 g einer 45 %ige wässrigen Natriumhydroxidlösung zugegeben und es wurde nochmals 12 h unter Rückfluß erhitzt. Die Lösung wurde auf Raumtemperatur abgekühlt und das Dioxan im Vakuum entfernt. Der Rückstand wurde mit wenig Wasser versetzt und mit Ethylacetat extrahiert. Die wässrige Phase wurde bei 0 °C mit konz. HCl auf pH 3 gestellt und der gebildete Niederschlag abfiltriert, man erhielt 5,04 g. Das Filtrat wurde zur Hälfte eingeengt und der ausfallende Feststoff abgesaugt, weitere 2,29 g konnten so isoliert werden.
Ausbeute: 7,33 g (72% d. Th.), logP¹⁾ (HCOOH) 0,50
¹H-NMR((CD₃)₂SO): 7,55 (m, 1H), 8,08 (s, 1H), 8,28 (m, 1H), 8,57 (m, 1H), 8,98 (s, 1H), 9,13 (m, 1H)

### Stufe 3: (Pyridin-3-yl)-1H-pyrazol-4-carbonylchloridhydrochlorid

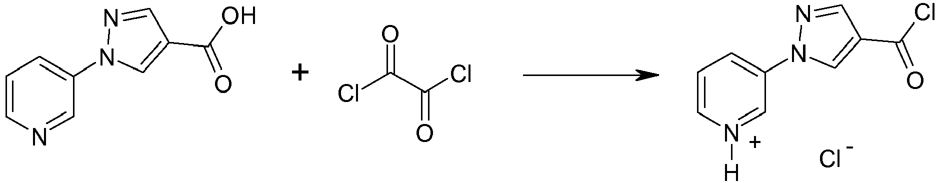

4,00 g (21,15 mmol) 1-(Pyridin-3-yl)-1H-pyrazol-4-carbonsäure wurden in ca 100 ml Dichlormethan vorgelegt und unter Argon mit 2 Tropfen Dimethylformamid versetzt. 7,783 g (61,32 mmol) Oxalsäuredichlorid wurden zugetropft und die Mischung nach Ende der Zugabe 1 Stunde bei Raumtemperatur gerührt. Die Lösung wurde eingeengt, der Rückstand mit ca 10 ml Toluol versetzt und eingengt. Man erhielt einen farblosen Feststoff, der sofort weiter umgesetzt wurde.

### Stufe 4: N-Ethyl-1-(pyridin-3-yl)-1H-pyrazol-4-carboxamid

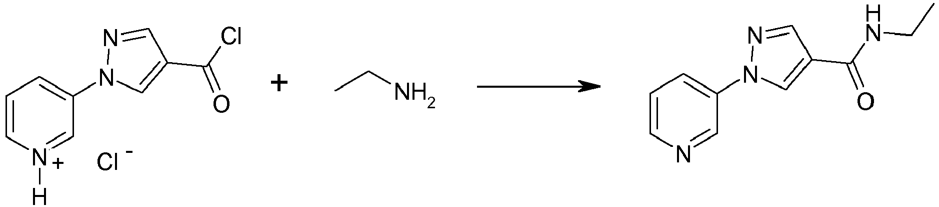

0,109 g (2,408 mmol) Ethylamin wurden in 20 ml Dioxan gelöst und 0,934 g (7,225 mmol) N,N-Diisopropylethylamin wurden unter Argon zugetropft. Eine Lösung von 0,500 g (2,408 mmol) Rohprodukt aus der Vorstufe in ca 40 ml Dioxan wurde zugegeben (leicht exotherm). Die Mischung wurde 12 h bei Raumtemperatur gerührt und anschließend eingeengt. Der Rückstand wurde mit Ethylacetat/Wasser versetzt und die wässrige Phase mit Ethylacetat extrahiert. Die organische Phase wurde mit Magnesiumsulfat getrocknet, dann eingeengt und mit Diethylether verrührt. Der Feststoff wurde abfiltriert.
Ausbeute: 0,240 g (46% d. Th.), logP¹⁾ (HCOOH) 0,64
¹H-NMR((CD₃)₂SO): 1,13 (t, 3H), 3,28 (q, 2H), 7,56 (m, 1H), 8,03 (NH, 1H), 8,15 (s, 1H), 8,21 (m, 1H), 8,55 (m, 1H), 8,90 (s, 1H), 9,08 (m, 1H)

### Beispiel B

### Stufe 1: Ethyl-3-methyl-1-(pyridin-3-yl)-1H-pyrazol-4-carboxylat

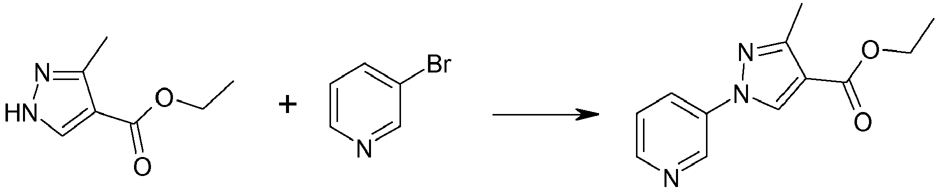

3,765 g (27,24 mmol) Kaliumcarbonat, 0,124 g (0,649 mmol) Kupferiodid, 0,296 g (2,595 mmol) Trans-N,N'-Dimethyl-1,2-cyclohexandiamin und 2,000 g (12,97 mmol) Pyrazol-3-methyl-4-carbonsäureethylester wurden in 5,0 ml (51,90 mmol) 3-Brompyridin unter Argon gelöst. Die Mischung wurde 24 Stunden auf 110 °C erhitzt und anschließend auf Raumtemperatur abgekühlt. Dichlormethan wurde zugegeben und der Feststoff abfiltriet. Das Filtrat wurde eingeengt und der Rückstand durch Chromatographie an Kieselgel gereinigt (Cyclohexan/Ethylacetat, 4:1).
Ausbeute: 2,18 g (73% d. Th.) logP¹⁾ (HCOOH) 1,83. Enthält 13% Regioisomer (logP¹⁾ (HCOOH) 1,63).
¹H-NMR((CD₃)₂SO): 1,31 (t, 3H), 2,46 (s, 3H), 4,27 (q, 2H), 7,53 (m, 1H), 8,26 (m, 1H), 8,55 (m, 1H), 8,96 (s, 1H), 9,11 (m, 1H)

### Stufe 2: 3-Methyl-1-(pyridin-3-yl)-1H-pyrazol-4-carbonsäure

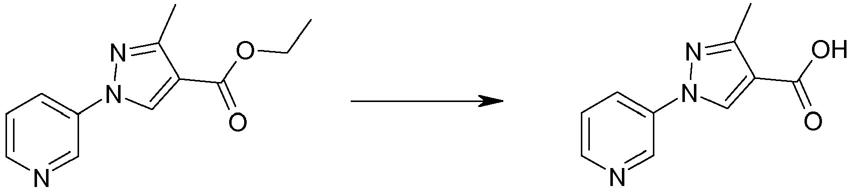

2,180 g (9,427 mmol) Ethyl-3-methyl-1-(pyridin-3-yl)-1H-pyrazol-4-carboxylat (mit ca 10% Regioisomer verunreinigt) wurden in ca 50 ml Dioxan gelöst und mit ca 15 ml Wasser und 2,011 g einer 45 %igen wässrigen Natriumhydroxidlösung versetzt. Die Mischung wurde 12 h bei Raumtemperatur gerührt. Da die Umsetzung nicht vollständig war, wurde die Reaktionsmischung zusätzlich 5 h unter Rückfluß erhitzt. Die Lösung wurde auf Raumtemperatur abgekühlt und das Dioxan im Vakuum entfernt. Der Rückstand wurde mit Wasser versetzt und mit Ethylacetat extrahiert. Die organische Phase wurde verworfen und die wässrige Phase bei 0 °C mit 1N HCl auf pH 3 gestellt und mit Ethylacetat extrahiert. Die organische Phase wurde mit Magnesiumsulfat getrocknet und eingeengt. Man erhielt 1,56 g der gewünschte Säure. Nach 12 h war ein Feststoff in der wässrigen Phase ausgefallen, er wurde abgesaugt und lieferte 0,090 g zusätzliches Produkt
Ausbeute: 1,65 g (86% d. Th.), logP¹⁾ (HCOOH) 0,77. Die Probe enthält 9% Regioisomers (logP¹⁾ (HCOOH) 0,53).
¹H-NMR((CD₃)₂SO): 2,45 (s, 3H), 7,52 (m, 1H), 8,23 (m, 1H), 8,53 (m, 1H), 8,91 (s, 1H), 9,01 (m, 1H)

### Stufe3: 3-Methyl-1-(pyridin-3-yl)-1H-pyrazol-4-carbonylchloridhydrochlorid

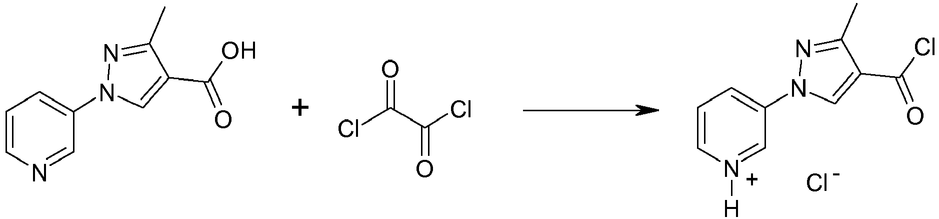

0,250 g (1,23 mmol) 3-Methyl-1-(pyridin-3-yl)-1H-pyrazol-4-carbonsäure wurden in ca 12 ml Dichloromethan vorgelegt und unter Argon mit 2 Tropfen Dimethylformamid versetzt. 0,453 g (3,56 mmol) Oxalsäuredichlorid wurden zugetropft und die Mischung nach Ende der Zugabe 1h bei Raumtemperatur gerührt. Die Lösung wurde eingeengt und der Rückstand mit ca 10 ml Toluol versetzt und eingeengt. Man erhielt einen farblosen Feststoff, der sofort weiter umgesetzt wurde.

### Stufe 4: 3-Methyl-1-(pyridin-3-yl)-N-(2,2,2-trifluorethyl)-1H-pyrazol-4-carboxamid

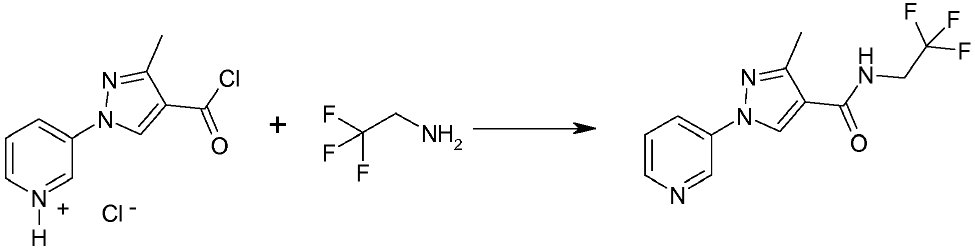

Zu 0,313 g (3,158 mmol) 2,2,2-Trifluor-1-aminoethan gelöst in 20 ml Dioxan wurden unter Argon 1,225 g (9,475 mmol) N,N-Diisopropylethylamin getropft. Eine Lösung von 0,700 g (3,158 mmol) 3-Methyl-1-(pyridin-3-yl)-1H-pyrazol-4-carbonylchloridhydrochlorid in ca 40 ml Dioxan wurde zugegeben (leicht exotherm). Die Mischung wurde 12 h bei Raumtemepratur gerührt und anschließend eingeengt. Der Rückstand wurde mit Dichlormethan/Wasser versetzt und die wässrige Phase mit Dichlormethan extrahiert. Die organische Phase wurde mit Magnesiumsulfat getrocknet, dann eingeengt und mit Diethylether verrührt. Der Feststoff wurde abfiltriert.
Ausbeute: 0,370 g (41% d. Th.), logP¹⁾ (HCOOH) 1,45
¹H-NMR((CD₃)₂SO): 2,45 (s, 3H), 4,05 (m, 2H), 7,55 (m, 1H), 8,14 (m, 1H), 8,43 (m, 1H), 8,55 (m, 1H), 8,95 (s, 1H), 9,00 (m, 1H)

### Beispiel C

### 1-(Pyridin-3-yl)-N-(2,2,2-trifluorethyl)-1H-pyrazol-4-carbothioamid

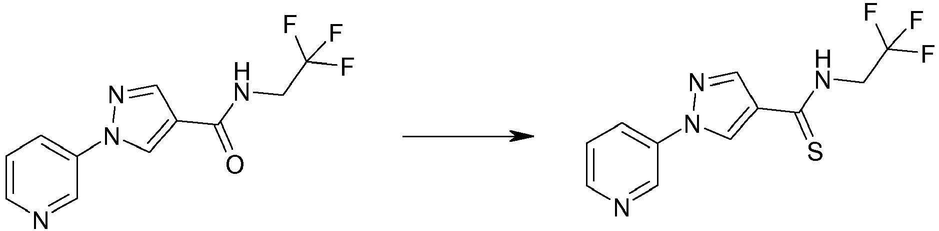

Eine Lösung von 1-(Pyridin-3-yl)-N-(2,2,2-trifluorethyl)-1H-pyrazol-4-carboxamid (100 mg, 0,37 mmol) in Toluol (10 ml) wurde mit 4-Methoxyphenyldithiophosphonsaeureanhydrid (82 mg, 0,20 mmol) versetzt. Die Reaktionsmischung wurde über Nacht bei 100 °C gerührt und anschließend abgekühlt. Eine Natriumhydrogencarbonat-Lösung wurde zugegeben und die Mischung zweimal mit Essigester extrahiert. Die vereinigten organischen Phasen wurden mit Magnesiumsulfat getrocknet und das Lösungsmittel im Vakuum entfernt. Der Rückstand wurde durch Chromatographie an Kieselgel (Cyclohexan, Essigester) gereinigt.
Ausbeute: 4,800 mg (4,5% d. Th.), logP¹⁾ (HCOOH) 1,97
¹H-NMR((CD₃)₂SO): 4,70 (m, 2H), 7,57 (m, 1H), 8,22 (m, 1H), 8,29 (s, 1H), 8,59 (m, 1H), 9,01 (s, 1H), 9,08 (m, 1H)

In der folgenden Tabelle sind weitere erfindungsgemäße Verbindungen aufgeführt, die analog bzw. gemäß den allgemeinen Angaben in der Beschreibung hergestellt wurden.

**Tabelle 1**

| **Bsp. Nr.** | **Formel** | **M⁺+1** | **logP (HCOOH)** | **¹H**-**NMR Daten (d₆-DMSO**, **Angaben in ppm)** |
|---|---|---|---|---|
| 1 (Beispiel A) | 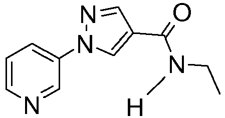 | 217,1 | 0,64 | 1,13 (t, 3H), 3,28 (q, 2H), 7,56 (m, 1H), 8,03 (NH, 1H), 8,15 (s, 1H), 8,21 (m, 1H), 8,55 (m, 1H), 8,90 (s, 1H), 9,08 (m, 1H) |
| 2 (Beispiel B) | 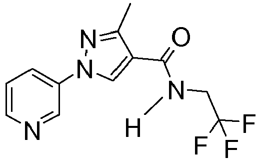 | 285 | 1,45 | 2,45 (s, 3H), 4,05 (m, 2H), 7,55 (m, 1H), 8,14 (m, 1H), 8,43 (m, 1H), 8,55 (m, 1H), 8,95 (s, 1H), 9,00 (m, 1H) |
| 3 | 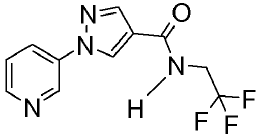 | 271,1 | 1,18 | 4,07 (m, 2H), 7,56 (m, 1H), 8,24 (m, 2H), 8,57 (m, 1H), 8,70 (bt, 1H), 9,01 (s, 1H), 9,09 (m, 1H) |
| 4 | 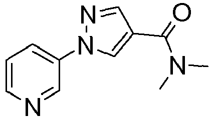 | 217,1 | 0,6 | 2,49 (s, 6H), 7,55 (m, 1H), 8,03 (s, 1H), 8,25 (m, 1H), 8,55 (m, 1H), 8,83 (s, 1H), 9,12 (m, 1H) |
| 5 | 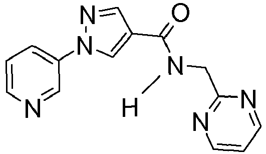 | 281,1 | 0,51 | 4,65 (d, 2H), 7,40-7,42 (t, 1H), 7,57-7,60 (m, 1H), 8,32-8,34 (m, 2H), 8,58 (m, 1H), 8,78 (m, 1H), 8,83-8,88 (m, 1H), 9,06 (s, 1H), 9,13 (m, 1H) |
| 6 | 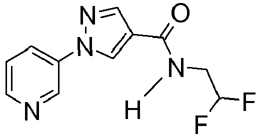 | 253,1 | 0,87 | 3,66 (m, 2H), 6,09 (tt, 1H), 7,57 (m, 1H), 8,22 (s, 1H), 8,24 (m, 1H), 8,46 (NH, 1H), 8,58 (m, 1H), 8,99 (s, 1H), 9,09 (m, 1H) |
| 7 | 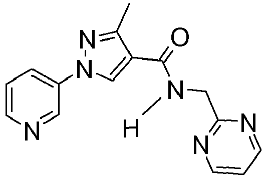 | 295,1 | 0,78 | 2,46 (s, 3H), 4,65 (m, 2H), 7,38 (t, 1H), 7,55 (m, 1H), 8,15 (m, 1H), 8,29 (m, 1H), 8,53 (m, 1H), 8,76 (m, 2H), 8,97 (s, 1H), 9,01 (m, 1H) |
| 8 | 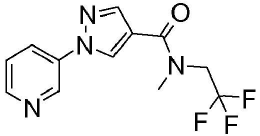 | 285,1 | 1,43 | 3,31 (s, 3H), 4,35 (q, 2H), 7,56 (m, 1H), 8,11 (m, 1H), 8,28 (m, 1H), 8,58 (m, 1H), 8,94 (s, 1H), 9,14 (m, 1H) |
| 9 | 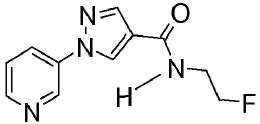 | 235,1 | 0,56 | 3,57 (dq, 2H), 4,52 (dt, 2H), 7,56 (m, 1H), 8,19 (s, 1H), 8,22 (m, 1H), 8,30 (NH, 1H), 8,57 (m, 1H), 8,95 (s,1H), 9,08 (m, 1H) |
| 10 | 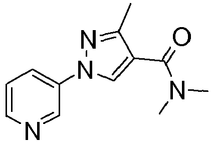 | 231,1 | 0,7 | 2,31 (s, 3H), 3,04 (s, 6H), 7,53 (m, 1H), 8,20 (m, 1H), 8,51 (m, 1H), 8,65 (s, 1H), 9,08 (m, 1H) |
| 11 | 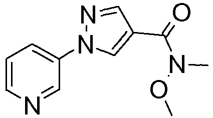 | 233,1 | 0,97 | 3,27 (s, 3H), 3,77 (s, 3H), 7,57 (m, 1H), 8,14 (s, 1H), 8,29 (m, 1H), 8,57 (m, 1H), 8,90 (s, 1H), 9,15 (m, 1H) |
| 12 (Beispiel C) | 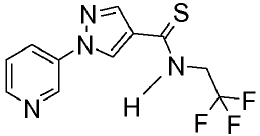 | 287,1 | 1,97 | 4,70 (m, 2H), 7,57 (m, 1H), 8,22 (m, 1H), 8,29 (s, 1H), 8,59 (m, 1H), 9,01 (s, 1H),9,08 (m, 1H) |
| 13 | 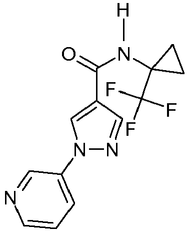 | 297 | 1,4 | 1,15 (m, 2H), 1,34 (m, 2H), 7,59 (m, 1H), 8,23-8,26 (m, 2H), 8,58 (m, 1H), 8,95 (s, 1H), 9,04 (s, 1H), 9,12 (m, 1H) |
| 14 | 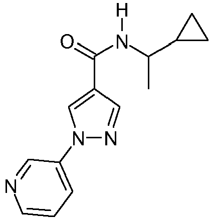 | 257,2 | 1,38 | 0,21 (m, 1H), 0,30 (m, 1H), 0,40 (m, 1H), 0,47 (m, 1H), 2,54 (s, 3H), 3,49 (m, 1H), 7,58 (m, 1H), 8,08 (m, 1H), 8,26 (m, 2H), 8,57 (m, 1H), 8,99 (s,1H), 9,10 (m, 1H) |
| 15 | 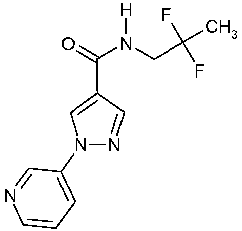 | 267,1 | 1,11 | 1,63 (t, 3H), 3,73 (m, 2H), 7,59 (m, 1H), 8,24-8,27 (m, 1H), 8,29 (s, 1H), 8,59 (m, 2H), 9,08 (s, 1H), 9,12 (m, 1H) |
| 16 | 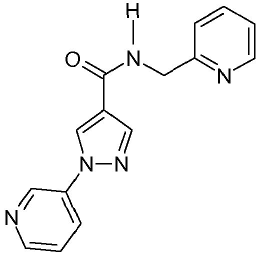 | 280,1 | 0,31 | 4,56 (m, 2H), 7,24-7,27 (m, 1H), 7,35 (m, 1H), 7,53-7,57 (m, 1H), 7,72-7,77 (m, 1H), 8,20-8,24 (m, 2H), 8,50 (m, 1H), 8,57 (m, 1H), 8,66 (m, 1H), 8,98 (s, 1H), 9,09 (m, 1H) |
| 17 | 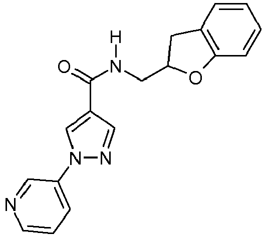 | 321,1 | 1,68 | 3,00 (m, 1H), 3,28 (m, 1H), 3,54 (m, 2H), 4,93 (m, 1H), 6,76 (m, 1H), 6,81 (m, 1H), 7,08 (m, 1H), 7,18 (m, 1H), 7,55 (m, 1H), 8,23 (m, 2H), 8,34 (NH, 1H), 8,56 (m, 1H), 8,95 (s, 1H), 9,08 (m, 1H) |
| 18 | 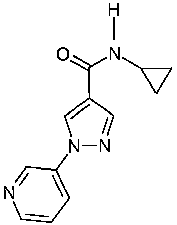 | 229,1 | 0,78 | 0,55 (m, 2H), 0,68 (m, 2H), 2,81 (m, 1H), 7,57 (m, 1H), 8,18 (s, 1H), 8,24 (m, 2H), 8,56 (m, 1H), 8,97 (s, 1H), 9,10 (m, 1H) |
| 19 | 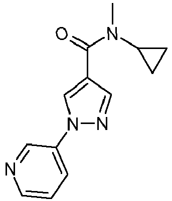 | 243,1 | 1,09 | 0,62 (m, 2H), 0,85 (m, 2H), 2,99 (s, 3H), 3,16 (m, 1H), 7,58 (m, 1H), 8,16 (s, 1H), 8,30 (m, 1H), 8,57 (m, 1H), 8,99 (s, 1H), 9,17 (m, 1H) |
| 20 | 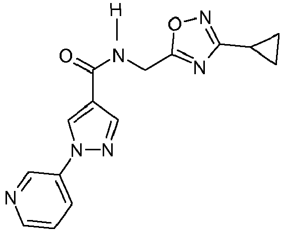 | 311,1 | 1,21 | 0,88 (m, 2H), 1,04 (m, 2H), 2,09 (m, 1H), 4,66 (d, 2H), 7,56 (m, 1H), 8,23 (m, 2H), 8,58 (m, 1H), 8,89 (NH, 1H), 8,98 (s, 1H), 9,09 (m, 1H) |
| 21 | 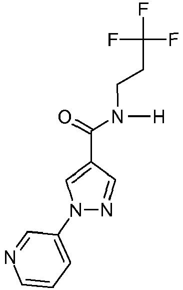 | 285 | 1,31 | 2,53 (m, 2H), 3,49 (m, 2H), 7,59 (m, 1H), 8,20 (s, 1H), 8,25 (m, 1H), 8,49 (m, 1H), 8,99 (s, 1H), 9,11 (m, 1H) |
| 22 | 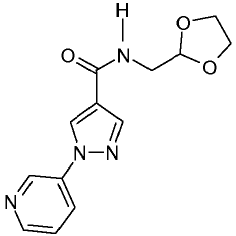 | 275,1 | 0,66 | 3,40 (m, 2H), 3,82 (m, 2H), 3,94 (m, 2H), 4,95 (t, 1H), 7,59 (m, 1H), 8,24-8,27 (m, 2H), 8,39 (m, 1H), 8,58 (m, 1H), 9,04 (s, 1H), 9,11 (m, 1H) |
| 23 | 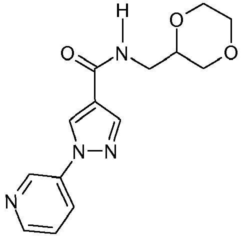 | 289,2 | 0,7 | 3,23-3,32 (m, 2H), 3,40-3,84 (m, 7H), 7,55 (m, 1H), 8,12 (NH, 1H), 8,18 (s, 1H), 8,21 (m, 1H), 8,57 (m, 1H), 8,94 (s, 1H), 9,07 (m, 1H) |
| 24 | 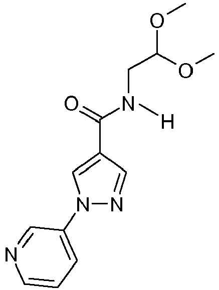 | 277,1 | 0,83 | 3,28-3,37 (m, 8H), 4,48 (m, 1H), 7,58 (m, 1H), 8,23 (s, 1H), 8,24-8,27 (m, 1H), 8,32 (m, 1H), 9,02 (s, 1H), 9,11 (m, 1H) |
| 25 | 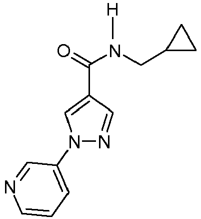 | 243,1 | 1,16 | 0,23 (m, 2H), 0,44 (m, 2H), 1,01 (m, 1H), 3,13 (t, 2H), 7,59 (m, 1H), 8,22-8,32 (m, 3H), 8,57 (m, 1H), 9,00 (s, 1H), 9,11 (m, 1H) |
| 26 | 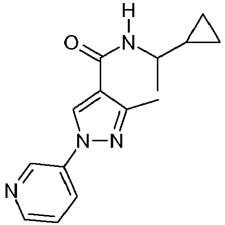 | 271,1 | 1,58/1,45 (Isomere) | 0,22 (m, 1H), 0,30-0,50 (m, 2H), 0,98 (m, 1H), 1,22 (d, 3H), 2,43 (s, 3H), 3,50 (m, 1H), 7,52 (m, 1H), 7,61 (NH, 1H), 8,11 (m, 1H), 8,51 (m, 1H), 8,88 (s, 1H), 9,02 (m, 1H) |
| 27 | 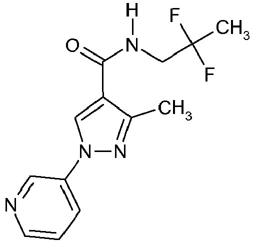 | 281,1 | 1,35 | 1,63 (t, 3H), 2,45 (s, 3H), 3,70 (m, 2H), 7,57 (m, 1H), 8,14 (m, 1H), 8,32 (m, 1H), 8,55 (m, 1H), 9,01 (m, 2H) |
| 28 | 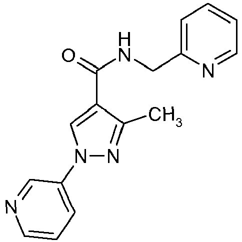 | 294,1 | 0,52 | 2,46 (s, 3H), 4,55 (d, 2H), 7,27 (m, 1H), 7,38 (m, 1H), 7,57 (m, 1H), 7,78 (m, 1H), 8,14 (m, 1H), 8,51-8,58 (m, 3H), 9,01 (m, 2H) |
| 29 | 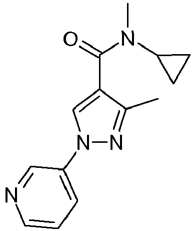 | 257,2 | 1,17 | 0,57 (m, 2H), 0,74 (m, 2H), 2,34 (s, 3H), 2,97 (s, 3H), 3,04 (m, 1H), 7,52 (m, 1H), 8,18 (m, 1H), 8,51(m, 1H), 8,75 (s, 1H), 9,07 (m, 1H) |
| 30 | | | | |
| 31 | 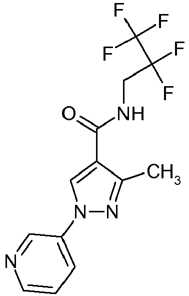 | 335 | 1,95 | 2,45 (s, 3H), 4,13 (m, 2H), 7,58 (m, 1H), 8,14 (m, 1H), 8,57 (m, 2H), 9,01 (m, 2H) |
| 32 | 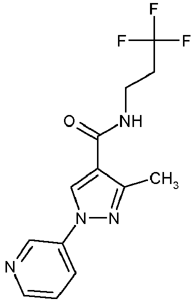 | 299,1 | 1,52 | 2,44 (s, 3H), 2,52 (m, 2H), 3,47 (m, 2H), 7,57 (m, 1H), 8,13 (m, 1H), 8,15 (m, 1H), 8,54 (m, 1H), 8,88 (s, 1H), 9,01 (m, 1H) |
| 33 | 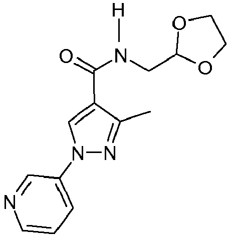 | 289,1 | 0,88 | 2,44 (s, 3H), 3,39 (m, 1H), 3,83 (m, 2H), 3,94 (m, 2H), 4,94 (m, 1H), 7,58 (m, 1H), 8,07 (m, 1H), 8,15 (m, 1H), 8,54 (m, 1H), 8,99 (m, 2H) |
| 34 | 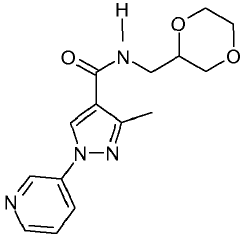 | 303,1 | 0,91 | 2,44 (s, 3H), 3,22-3,40 (m, 5H), 3,47 (m, 1H), 3,58 (m, 2H), 3,75 (m, 1H), 7,56 (m, 1H), 8,03 (m, 1H), 8,15 (m, 1H), 8,54 (m, 1H), 8,95 (s, 1H), 9,13 (m, 1H) |
| 35 | 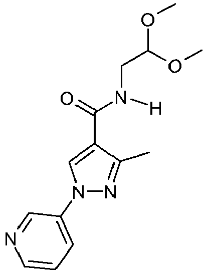 | 291,1 | 1,04 | 2,44 (s, 3H), 3,32 (s, 6H), 3,34 (m, 2H), 4,47 (t, 1H), 7,53 (m, 1H), 7,77 (NH, 1H), 8,11 (m, 1H), 8,53 (m, 1H), 8,90 (s, 1H), 8,99 (m, 1H) |
| 36 | 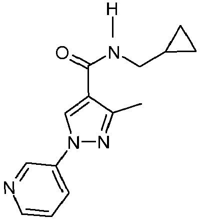 | 257,2 | 1,32 | 0,22-0,25 (m, 2H), 0,43-0,48 (m, 2H), 1,01 (m, 1H), 2,44 (s, 3H), 2,52 (m, 2H), 7,52-7,55 (m, 1H), 7,82 (NH, 1H), 8,11-8,14 (m, 1H), 8,53 (m, 1H), 8,87 (s, 1H), 9,00 (m, 1H) |
| 37 | 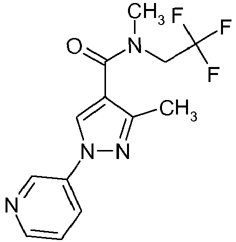 | 299,1 | 1,57 | 2,33 (s, 3H), 8,29 (s, 3H), 4,37 (q, 2H), 7,55 (m, 1H), 8,24 (m, 1H), 8,53 (m, 1H), 8,54 (s, 1H), 9,11 (m, 1H) |
| 38 | 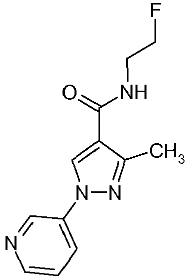 | 249,1 | 0,87 | 2,45 (s, 3H), 3,54 (m, 2H), 4,53 (m, 2H), 7,55 (m, 1H), 8,15 (m, 1H), 8,23 (m, 1H), 8,55 (m, 1H), 9,00 (m, 1H) |
| 39 | 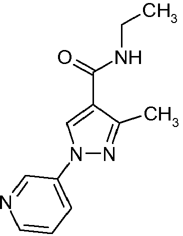 | 231,1 | 0,91 | 1,12 (t, 3H), 2,44 (s, 3H), 3,27 (m, 2H), 7,55 (m, 1H), 7,94 (m, 1H), 8,13 (m, 1H), 8,54 (m, 1H), 9,01 (m, 1H) |
| 40 | 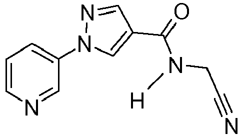 | 228,1 | 0,52 | 4,30 (d, 2H), 7,58 (m, 1H), 8,20 (s, 1H), 8,23 (m, 1H), 8,58 (m, 1H), 8,84 (NH, 1H), 8,99 (s, 1H), 9,10 (m, 1H) |
| 41 | 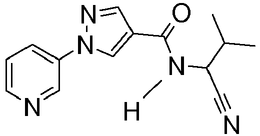 | 270,1 | 1,4 | 1,02 (d, 3H), 1,10 (d, 3H), 2,15 (m, 1H), 4,80 (t, 1H), 7,57 (m, 1H), 8,22 (m, 1H), 8,25 (s, 1H), 8,58 (m, 1H), 8,74 (NH, 1H), 9,03 (s,1H), 9,10 (m,1H) |
| 42 | 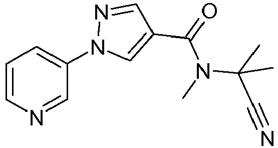 | 270,1 | 1,09 | 1,74 (s, 6H), 3,16 (s, 3H), 7,56 (m, 1H), 8,09 (s, 1H), 8,26 (m, 1H), 8,57 (m, 1H), 8,93 (s, 1H), 9,13 (m, 1H) |
| 43 | 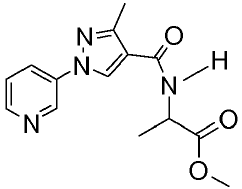 | 289,1 | 1,14 | 1,39 (d, 3H), 2,43 (s, 3H), 3,66 (s, 3H), 4,48 (qu, 1H), 7,55 (m, 1H), 8,12 (m, 2H), 8,53 (m, 1H), 8,95 (s, 2H), 9,00 (m, 1H) |
| 44 | 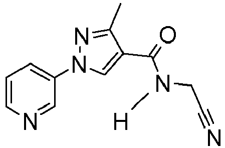 | 242,1 | 0,79 | 2,45 (s, 3H), 4,28 (d, 2H), 7,55 (m, 1H), 8,13 (m, 1H), 8,54 (m, 2H), 8,88 (s,1H), 9,00 (m,1H) |
| 45 | 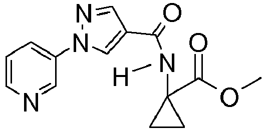 | 287,1 | 0,84 | 1,16 (m, 2H), 1,45 (m, 2H), 3,61 (s, 3H), 7,56 (m, 1H), 8,18 (s, 1H), 8,22 (m, 1H), 8,57 (m, 1H), 8,68 (NH, 1H), 8,92 (s, 1H), 9,08 (m, 1H) |
| 46 | 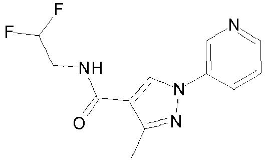 | 267,1 | 1,12 | 2,45(s,3H), 3,60-3,70(m,2H), 6,11(m,1H), 7,55-7,59(m,1H), 8,13-8,16(m,1H), 8,34-8,37(t,1H), 8,55(m,1H), 8,98(s,1H), 9,00(m,1H) |
| 47 | 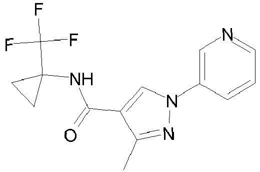 | 311,2 | 1,63 | 1,12-1,14(m,2H), 1,30-1,35(m,2H), 2,43(s,3H), 7,55-7,59(m,1H), 8,11-8,14(m,1H), 8,54-8,56(m,1H), 8,71(s,1H), 8,96(s,1H), 8,98-8,99(m,1H) |
| 48 | 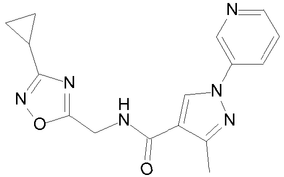 | 325,2 | 1,43 | 0,86-0,90(m,2H), 1,03-1,08(m,2H), 2,06-2,12(m,1H), 2,44(s,3H), 4,64(d,2H), 7,53-7,57(m,1H), 8,12-8,15(m,1H), 8,53-8,55(m,1H), 8,60(t,1H), 8,90(s,1H), 8,99-9,00(m,1H) |
| 49 | 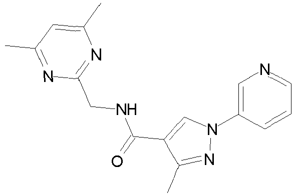 | 232,1 | 1,2 | 2,40(s,6H), 2,47(s,3H), 4,55(d,2H), 7,11(s,1H), 7,52-7,56(m,1H), 8,12-8,16(m,1H), 8,19(broad, NH), 8,52-8,54(m,1H), 8,95(s,1H), 9,01-9,02(m,1H) |
| 50 | 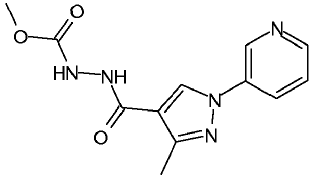 | 276,2 | 0,52 | 2,44(s,3H); 3,63(s,3H); 7,53-7,59(m,1H); 8,13-8,16(m,1H); 8,55-8,57(m,1H); 8,92(s,1H); 9,01(m,1H); 9,16(s,1H) |
| 51 | 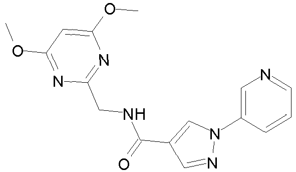 | 341,1 | 1,37 | 3,87(s,6H), 4,47(d,2H), 6,07(s,1H), 7,53-7,57(m,1H), 8,21-8,24(m, 2H), 8,48(broad,NH), 8,55-8,57(m,1H), 8,98-8,99(m,1H), 9,09-9,10(m,1H) |
| 52 | 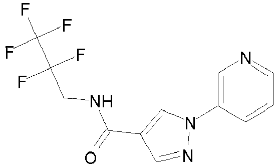 | 321,0 | 1,69 | 4,10-4,20(m,2H), 7,56-7,60(m,1H), 8,25-8,29(m,1H), 8,30(s,1H), 8,58-8,60(m,1H), 8,84-8,87(m,1H), 9,10(s,1H), 9,12-9,19(m,1H) |
| 53 | 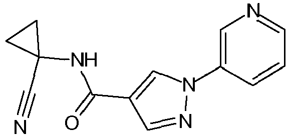 | 254,1 | 0,76 | 1,25-1,29(m,2H), 1,53-1,56(m,2H), 7,54-7,58(m,1H), 8,17(s,1H), 8,21-8,24(m,1H), 8,57-8,58(m,1H), 8,96-8,97(m,2H), 9,07-9,08(m,1H) |
| 54 | 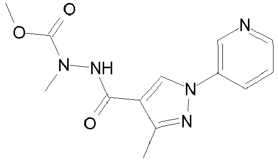 | 290,1 | 0,79 | 2,36(s,3H), 3,12(s,3H), 3,58(s,3H), 7,58(m,1H), 8,13(m,1H), 8,56(m,2H), 8,65(m,1H) |
| 55 | 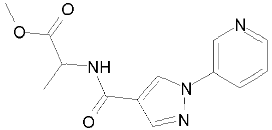 | 275,1 | 0,91 | 1,40(s,3H), 3,66(s,3H), 4,51(m,1H), 7,54-7,58(m,1H), 8,21-8,24(m,2H), 8,39(d,NH), 8,56-8,58(m,1H), 8,98-8,99(m,1H), 9,08-9,09(m,1H) |
| 56 | 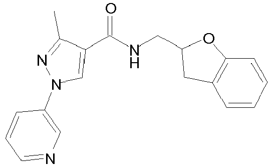 | 335,1 | 1,9 | 2,42(s,3H), 2,90-3,10(m,1H), 3,30-3,40(m,1H), 3,50-3,60(m,2H), 4,88-5,00(m,1H), 6,20-6,40(m,2H), 7,05-7,15(m,1H), 7,20-7,25(m,1H), 7,52-7,58(m,1H), 8,10-8,20(m,1H), 8,27-8,33(m,1H), 8,51-8,54(m,1H), 8,91-8,94(m,1H), 8,98-9,03(m,1H) |
| 57 | 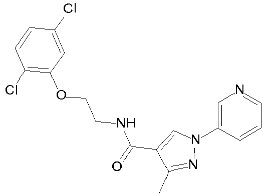 | 391,0 | 2,56 | 2,45(s,3H), 3,63(q,2H), 4,25(t,2H), 7,00-7,03(m,1H), 7,28-7,29(m,1H), 7,42(d,1H), 7,52-7,57(m,1H), 7,97-8,00(m,NH), 8,10-8,13(m,1H), 8,52-8,54(m,1H), 8,84(s,1H), 8,98-8,99(m,1H) |

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### 2) Messung der NMR-Spektren

Die NMR-Spektren wurden
a) mit einem Bruker Avance 400, ausgestattet mit einem Durchflussprobenkopf (60 µl Volumen), bestimmt. Als Lösungsmittel wurden CD₃CN oder d₆-DMSO verwendet, wobei als Referenz Tetramethylsilan (0.00 ppm) eingesetzt wurde.
b) mit einem Bruker Avance II 600 bestimmt. Als Lösungsmittel wurden CD₃CN oder d₆-DMSO verwendet, wobei als Referenz Tetramethylsilan (0.00 ppm) eingesetzt wurde.

Die Aufstpaltung der Signale wurde wie folgt beschrieben: s (Singulett), d (Duplett), t (Triplett), q (Quartett), quin (Quintett), m (Multiplett).

### Biologische Beispiele

### Beispiel 1: Boophilus microplus - Test (Injektion)

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigt z.B. die folgende Verbindung der Herstellungsbeispiele eine Wirkung von 100% bei einer Aufwandmenge von 20µg/Tier: 9

### Beispiel 2: Myzus-Test (Spritzbehandlung)

| | | |
|---|---|---|
| Lösungsmittel: | 78 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele Wirkung von 80% bei einer Aufwandmenge von 500g/ha: 12

Bei diesem Test zeigen z. B. die folgenden Verbindungen der Herstellungsbeispiele Wirkung von 90% bei einer Aufwandmenge von 500g/ha: 4, 30, 52, 53, 55

Bei diesem Test zeigen z. B. die folgenden Verbindungen der Herstellungsbeispiele Wirkung von 100% bei einer Aufwandmenge von 500g/ha: 1, 2, 3, 5, 6, 7, 8, 9, 10, 13, 14, 15, 16, 18, 19, 20, 21, 22, 23, 24, 25, 26, 27, 28, 29, 31, 32, 33, 34, 35, 36, 37, 38, 39, 40, 41, 42, 43, 45, 46, 47, 48, 49, 50, 54, A-4

### Beispiel 3: Phaedon-Test (Spritzbehandlung)

| | | |
|---|---|---|
| Lösungsmittel: | 78,0 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigt z. B. die folgende Verbindung der Herstellungsbeispiele Wirkung von 100% bei einer Aufwandmenge von 500g/ha: 3

## Patentansprüche

1. Verbindungen der Formel (I), 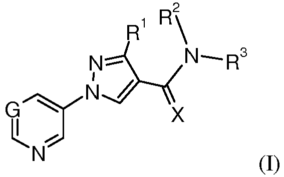 worin
G für N, CH, C-Halogen, C-Nitro, C-Cyano, C-C₁-C₆-Alkyl, C-C₁-C₆-Haloalkyl, C-C₃-C₆-Cycloalkyl, C-C₁-C₆-Alkoxy, C-C₁-C₆-Haloalkoxy steht, R¹ für Wasserstoff, C₁-C₆-Alkyl, C₁-C₆-Haloalkyl, C₃-C₆-Cycloalkyl, Halogen, Cyano, C₁-C₆-Alkoxy, C₁-C₆-Haloalkoxy, Amino, C₁-C₆-Alkylanüno, Di-(C₁-C₆)-alkylamino oder C₁-C₆-Thioalkyl steht,
X für Sauerstoff oder Schwefel steht,
R² für einen Rest aus der Reihe Wasserstoff, C₁-C₆-Alkyl, C₁-C₆-Halogenalkyl, C₁-C₄-Alkoxy, C₁-C₄-Halogenalkoxy, C₂-C₄-Alkenyl, C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl und gegebenenfalls durch Halogen substituiertes C₃-C₆-Cycloalkylcarbonyl steht,
R³ für einen Rest aus der Reihe Wasserstoff, C₁-C₆-Alkyl, C₁-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylthio-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, gegebenenfalls durch Halogen substituiertes C₁-C₆-Alkoxycarbonyl-C₁-C₆-alkyl, gegebenenfalls am Stickstoff durch C₁-C₆-Alkyl substituiertes C₁-C₆-Alkoxycarbonylamino, C₂-C₄-Alkinyloxy, C₂-C₄-Alkinyloxycarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes und gegebenenfalls benzokondensiertes Heterocyclyl-C₁-C₆-alkyl, gegebenenfalls durch Halogen, Cyano, C₁-C₆-Alkyl, C₁-C₆-Halogenalkyl, C₁-C₆-Alkoxy oder C₁-C₆-Halogenalkoxy substituiertes Aryl-C₁-C₆-alkyl, gegebenenfalls im Arylteil durch Halogen substituiertes Aryloxy-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C6-alkyl, C₁-C₆-Alkylcarbonyl, C₁-C₆-Alkoxycarbonyl oder Aminocarbonyl substituiertes Hetaryl-C₁-C₆-alkyl, und NR⁴R⁵ steht, worin R⁴ und R⁵ unabhängig voneinander für einen Rest aus der Reihe Wasserstoff, C₁-C₆-Alkyl, C₁-C₆-Halogenalkyl, C₃-C₆-Cycloyalkyl, C₁-C₆-Alkoxy, C₁-C₆-Alkylcarbonyl und C₁-C₆-Alkoxycarbonyl stehen,
oder
R² und R³ gemeinsam mit dem Stickstoffatom, an das sie gebunden sind, einen gegebenenfalls durch C₁-C₆-Alkyl oder C₁-C₆-Alkoxy substituierten und gegebenenfalls ein oder zwei weitere Heteroatome aus der Reihe Sauerstoff, Stickstoff und Schwefel enthaltenden 3 bis 7 gliedrigen Ring bilden und worin
Halogen (bzw. Halo) ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl,
Hetaryl (auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl und
Heterocyclyl (auch als Teil einer größeren Einheit, wie beispielsweise Heterocyclylalkyl) ausgewählt aus der Reihe 2-Tetrahydrofuranyl, 3-Tetrahydrofuranyl, 2,3-Dihydro-1-benzofur-2-yl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 1,4-Dioxan-2-yl, 1,3-Dioxolan-2-yl, 1,3-Dioxolan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

2. Verbindungen der Formel (I) gemäß Anspruch 1, worin
G für N, CH, C-Halogen, C-Nitro, C-Cyano, C-C₁-C₄-Alkyl, C-C₁-C₄-Haloalkyl, C-C₃-C₆-Cycloalkyl, C-C₁-C₄-Alkoxy, C-C₁-C₄-Haloalkoxy steht, R¹ für Wasserstoff, C₁-C₄-Alkyl, C₁-C₄-Haloalkyl, C₃-C₆-Cycloalkyl, Halogen, Cyano, C₁-C₄-Alkoxy, C₁-C₄-Haloalkoxy, Amino, C₁-C₄-Alkylamino, Di-(C₁-C₄)-alkylamino oder C₁-C₄-Thioalkyl steht,
X für Sauerstoff oder Schwefel steht,
R² für einen Rest aus der Reihe Wasserstoff, C₁-C₄-Alkyl, C₁-C₄-Halogenalkyl, C₁-C₄-Alkoxy, C₂-C₃-Alkenyl, C₁-C₂-Alkoxy-C₁-C₂-alkyl, C₁-C₂-Alkylcarbonyl, C₁-C₂-Alkoxycarbonyl und jeweils gegebenenfalls durch Halogen substituiertes Cyclopropylcarbonyl, Cyclopentylcarbonyl oder Cyclohexylcarbonyl steht,
R³ für einen Rest aus der Reihe Wasserstoff, C₁-C₄-Alkyl, C₁-C₅-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylthio-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, gegebenenfalls durch Halogen substituiertes C₁-C₄-Alkoxycarbonyl-C₁-C₄-alkyl, gegebenenfalls am Stickstoff durch C₁-C₄-Alkyl substituiertes C₁-C₄-Alkoxycarbonylamino, C₂-C₄-Alkinyloxy, C₂-C₄-Alkinyloxycarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes und gegebenenfalls benzokondensiertes Heterocyclyl-C₁-C₄-alkyl, gegebenenfalls durch Halogen, Cyano, C₁-C₄-Alkyl, C₁-C₄-Halogenalkyl, C₁-C₄-Alkoxy oder C₁-C₄-Halogenalkoxy substituiertes Aryl-C₁-C₄-alkyl, gegebenenfalls im Arylteil durch Halogen substituiertes Aryloxy-C₁-C₄-alkyl und gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl steht
oder
R² und R³ gemeinsam mit dem Stickstoffatom, an das sie gebunden sind, einen gegebenenfalls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring bilden,und worin
Halogen (bzw.Halo) ausgewählt ist aus der Reihe Fluor, Chlor, Brom und Iod, Aryl (auch als Teil einer größeren Einheit, wie beispielsweise Arylalkyl) ausgewählt ist aus der Reihe Phenyl, Naphthyl, Anthryl, Phenanthrenyl,
Hetaryl (auch als Teil einer größeren Einheit, wie beispielsweise Hetarylalkyl) ausgewählt ist aus der Reihe Pyrimidyl, Oxadiazolyl, Oxazolyl, Pyrazinyl, Imidazolyl, Thiazolyl und Furanyl und
Heterocyclyl (auch als Teil einer größeren Einheit, wie beispielsweise Heterocyclylalkyl) ausgewählt ist aus der Reihe 2-Tetrahydrofuranyl, 3-Tetrahydrofuranyl, 2,3-Dihydro-1-benzofur-2-yl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazofin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 1,4-Dioxan-2-yl, 1,3-Dioxolan-2-yl, 1,3-Dioxolan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl.

3. Verbindungen der Formel (I) gemäß Anspruch 1, worin
G für N, CH, C-Halogen, C-Cyano, C-CH₃, C-CF₃, C-Cyclopropyl, C-OCH₃, C-OCF₃ steht,
R¹ für Wasserstoff, Methyl, Trifluoromethyl, Cyclopropyl, Halogen, Cyano, Methoxy, Trifluoromethoxy, Amino, Methylamino, Dimethylamino steht,
X für Sauerstoff oder Schwefel steht,
R² für einen Rest aus der Reihe Wasserstoff, Methyl, Ethyl, CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, Methoxy, Ethoxy, Vinyl, CH₂OCH₃, CH₂OCH₂CH₃, Methylcarbonyl, Ethylcarbonyl, Methoxycarbonyl, Ethoxycarbonyl, Cyclopropylcarbonyl und Fluorcyclpropylcarbonyl steht,
R³ für einen Rest aus der Reihe Wasserstoff, Methyl, Ethyl, CH₂CF₃, CH₂CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, CH(CN)CH(CH₃)₂, CH₂CN, CH₂CH₂CN, Vinyl, C(CH₃)₂CCH, CH₂CCCH₃, Methoxy, Ethoxy, CH₂CH(OCH₃)₂, CH₂CH(CH₃)(OCH₃), CH₂C(CH₃)₂(OCH₃), CH(CH₃)CH(OCH₃)₂, CH₂C(CH₃)(OCH₃)₂, C(CH₃)₂CH₂SCH₃, CH₂CH₂SCH₃, CHCH₃CH₂SCH₃, gegebenenfalls durch Halogen substituiertes C₁-C₄-Alkylcarbonyl-C₁-C₄-alkyl, CH₃SO₂CH₂C(CH₃)₂, CH₃SO₂CH₂CHCH₃, Propargyloxy, Cyclopropyl, Cyanocyclopropyl, Fluorcyclopropyl, Trifluormethylcyclopropyl, Trifluormethylcyclohexyl, Methoxycarbonylcyclopropyl, Fluorcyclopropylcarbonyl, Cyclopropylmethyl, 1-Cyclopropylethyl, Cyclohexylmethyl, 1-Cyano-1-cyclopropyl-eth-1-yl, 1,3-Dioxolan-2-ylmethyl, 4-Methyl-1,3-Dioxolan-2-ylmethyl, Tetrahydrofurylmethyl, Tetrahydropyranylmethyl, 2,2-Dimethyl-1,3-dioxolan-5-yl-methyl, 2-Methyltetrahydrofur-2-yl-methyl, αα-Methyl-3,5-dimethyltriazol-1-yl-ethyl, 1,5-Dimethyl-1,3-oxazol-4-yl-methyl, gegebenenfalls durch Halogen, Cyano, Methyl, Ethyl, Methoxy oder Ethoxy substituiertes Benzyl, für Pyrimidylmethyl, Pyridylmethyl, α-Methyl-pyrimidylmethyl, Oxadiazolylmethyl, Oxazolylmethyl, 5-Methyl-pyrazin-2-yl, α-Methyl-pyrid-2-yl-methyl, 4-Brom-pyrimid-2-yl-methyl, Imidazolylmethyl, 2-Methylpyrimid-4-yl-methyl, 6-Chlor-pyridin-3-yl-methyl, 4,6-Dimethylpyrimid-2-yl-methyl, Thiazolylmethyl, Furanylmethyl, 1,5-Dimethylpyrazol-3-yl-methyl, 4-lod-pyrimid-2-yl-methyl, 3-Cyclopropyl-1,2,4-oxdiazol-5-yl-methyl, 2-Ethyl-pyrimid-6-yl-methyl, 6-Brom-pyrid-2-yl-methyl, (2,3-Dihydro-1-benzofur-2-yl)methyl, (1,4-Dioxan-2-yl)methyl, 1,3-Dioxolan-2-ylmethyl, Methoxycarbonylethyl (Methylalaninat), Methoxycarbonylamino, 4,6-Dimethoxypyrimid-2-ylmethyl, 2-(2,5-Dichlorphenoxy)ethyl und N-Methyl-N-Methoxycarbonyl-amino steht,
oder
R² und R³ gemeinsam mit dem Stickstoffatom, an das sie gebunden sind, einen gegebenenfalls durch C₁-C₄-Alkyl oder C₁-C₄-Alkoxy substituierten und gegebenenfalls ein oder zwei weitere Heteroatome aus der Reihe Sauerstoff, Stickstoff und Schwefel enthaltenden 3 bis 7 gliedrigen Ring bilden.

4. Verfahren zur Herstellung von Verbindungen der Formel (I) gemäß Anspruch 1, **dadurch gekennzeichnet, dass** man
Verbindungen der Formel (II) 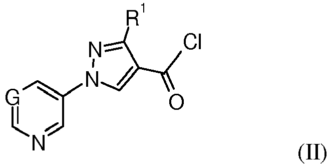 in welcher
G und R¹ die im Anspruch 1 genannten Bedeutungen haben
mit Verbindungen der Formel (III) 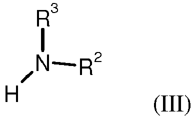 in welcher
R² und R³ die im Anspruch 1 genannten Bedeutungen haben,
gegebenenfalls in Gegenwart eines geeigneten Verdünnungsmittels und gegebenenfalls in Gegenwart eine Base umsetzt und gegebenenfalls die nach diesem Verfahren erhältlichen Verbindungen der Formel (I), in denen X für Sauerstoff steht, durch Umsetzung mit einem Schwefelungsreagenz in Verbindungen der Formel (I) überführt, in denen X für Schwefel steht.

5. Mittel, **gekennzeichnet durch** einen Gehalt von mindestens einer Verbindung der Formel (I) gemäß Ansprüchen 1 bis 3 und üblichen Streckmitteln und/oder oberflächenaktiven Substanzen.

6. Verfahren zum Bekämpfen von Schädlingen **dadurch gekennzeichnet, dass** man eine Verbindung der Formel (I) gemäß Ansprüchen 1 bis 3 oder ein Mittel gemäß Anspruch 5 auf die Schädlinge und/oder ihren Lebensraum einwirken lässt.

7. Verwendung von Verbindungen der Formel (I) gemäß Ansprüchen 1 bis 3 oder von Mitteln gemäß Anspruch 5 zum Bekämpfen von Schädlingen.

## Claims

1. Compounds of the formula (I) 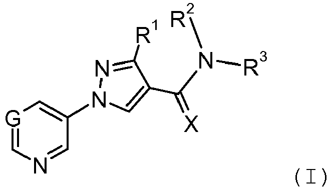 in which
G is N, CH, C-halogen, C-nitro, C-cyano, C-C₁-C₆-alkyl, C-C₁-C₆-haloalkyl, C-C₃-C₆-cycloalkyl, C-C₁-C₆-alkoxy, C-C₁-C₆-haloalkoxy,
R¹ is hydrogen, C₁-C₆-alkyl, C₁-C₆-haloalkyl, C₃-C₆-cycloalkyl, halogen, cyano, C₁-C₆-alkoxy, C₁-C₆-haloalkoxy, amino, C₁-C₆-alkylamino, di (C₁-C₆)-alkylamino or C₁-C₆-thioalkyl,
X is oxygen or sulphur,
R² is a radical from the group of hydrogen, C₁-C₆-alkyl, C₁-C₆-haloalkyl, C₁-C₄-alkoxy, C₁-C₄-haloalkoxy, C₂-C₄-alkenyl, C₁-C₄-alkoxy-C₁-C₄-alkyl, optionally halogen-substituted C₁-C₄-alkylcarbonyl, optionally halogen-substituted C₁-C₄-alkoxycarbonyl and optionally halogen-substituted C₃-C₆-cycloalkylcarbonyl,
R³ is a radical from the group of hydrogen, C₁-C₆-alkyl, C₁-C₆-haloalkyl, cyano-C₁-C₆-alkyl, C₂-C₄-alkenyl, C₂-C₄-haloalkenyl, C₂-C₄-alkynyl, C₂-C₄-haloalkynyl, C₁-C₆-alkoxy, C₁-C₆-haloalkoxy, optionally halogen-substituted C₁-C₆-alkoxy-C₁-C₆-alkyl, optionally halogen-substituted bis(C₁-C₆-alkoxy)-C₁-C₆-alkyl, optionally halogen-substituted C₁-C₆-alkylthio-C₁-C₆-alkyl, optionally halogen-substituted C₁-C₄-alkylcarbonyl-C₁-C₄-alkyl, optionally halogen-substituted C₁-C₆-alkylsulphinyl-C₁-C₆-alkyl, optionally halogen-substituted C₁-C₆-alkylsulphonyl-C₁-C₆-alkyl, optionally halogen-substituted C₁-C₆-alkoxycarbonyl-C₁-C₆-alkyl, C₁-C₆-alkoxycarbonylamino optionally C₁-C₆-alkylsubstituted on the nitrogen, C₂-C₄-alkynyloxy, C₂-C₄-alkynyloxycarbonyl, optionally halogen-, cyano-, C₁-C₆-alkyl-, C₁-C₆-haloalkyl-, C₁-C₆-alkoxy-, C₁-C₆-haloalkoxy-, C₁-C₆-alkoxycarbonyl-, C₁-C₆-haloalkoxycarbonyl- or hetaryl-substituted (where hetaryl is itself optionally substituted by C₁-C₆-alkyl or halogen) C₃-C₆-cycloalkyl, optionally halogen-, cyano-, C₁-C₆-alkyl-, C₁-C₆-haloalkyl-, C₁-C₆-alkoxy-, C₁-C₆-haloalkoxy-, C₁-C₆-alkoxycarbonyl-, C₁-C₆-haloalkoxycarbonyl- or hetaryl-substituted (where hetaryl is itself optionally substituted by C₁-C₆-alkyl or halogen) C₃-C₆-cycloalkylcarbonyl, optionally halogen-, cyano-, C₁-C₆-alkyl-, C₁-C₆-haloalkyl-, C₁-C₆-alkoxy-, C₁-C₆-haloalkoxy-, C₁-C₆-alkoxycarbonyl-, C₁-C₆-haloalkoxycarbonyl- or hetaryl-substituted (where hetaryl is itself optionally substituted by C₁-C₆-alkyl or halogen) C₃-C₆-cycloalkyl-C₁-C₆-alkyl, optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-alkylsulphonyl-, C₁-C₆-haloalkylsulphinyl-, C₁-C₆-haloalkylsulphonyl-, amino-, C₁-C₆-alkylamino-, di (C₁-C₆-alkyl)amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted and optionally benzofused heterocyclyl-C₁-C₆-alkyl, optionally halogen-, cyano-, C₁-C₆-alkyl-, C₁-C₆-haloalkyl-, C₁-C₆-alkoxy- or C₁-C₆-haloalkoxy-substituted aryl-C₁-C₆-alkyl, optionally in the aryl moiety, halogen-substituted aryloxy-C₁-C₆-alkyl, optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-alkylsulphonyl-, C₁-C₆-haloalkylsulphinyl-, C₁-C₆-haloalkylsulphonyl-, amino-, C₁-C₆-alkylamino-, di (C₁-C₆-alkyl)amino-, C₁-C₆-alkylcarbonylamino-, C₁-C₆-alkoxycarbonyl-amino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted hetaryl-C₁-C₆-alkyl, and NR⁴R⁵ in which R⁴ and R⁵ are each independently a radical from the group of hydrogen, C₁-C₆-alkyl, C₁-C₆-haloalkyl, C₃-C₆-cycloalkyl, C₁-C₆-alkoxy, C₁-C₆-alkylcarbonyl and C₁-C₆-alkoxycarbonyl,
or
R² and R³ together with the nitrogen atom to which they are bonded form an optionally C₁-C₆-alkyl- or C₁-C₆-alkoxy-substituted 3- to 7-membered ring which optionally contains one or two further heteroatoms from the group of oxygen, nitrogen and sulphur and in which
halogen (or halo) is selected from the group of fluorine, chlorine, bromine and iodine,
aryl (including as part of a larger unit, for example arylalkyl) is selected from the group of phenyl, naphthyl, anthryl, phenanthrenyl,
hetaryl (including as part of a larger unit, for example hetarylalkyl) is selected from the group of furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl and
heterocyclyl (including as part of a larger unit, for example heterocyclylalkyl) is selected from the group of 2-tetrahydrofuranyl, 3-tetrahydrofuranyl, 2,3-dihydro-1-benzofur-2-yl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,3-dihydrothien-2-yl, 2,3-dihydrothien-3-yl, 2,4-dihydrothien-2-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 1,4-dioxan-2-yl, 1,3-dioxolan-2-yl, 1,3-dioxolan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl.

2. Compounds of the formula (I) according to Claim 1, in which
G is N, CH, C-halogen, C-nitro, C-cyano, C-C₁-C₄-alkyl, C-C₁-C₄-haloalkyl, C-C₃-C₆-cycloalkyl, C-C₁-C₄-alkoxy, C-C₁-C₄-haloalkoxy,
R¹ is hydrogen, C₁-C₄-alkyl, C₁-C₄-haloalkyl, C₃-C₆-cycloalkyl, halogen, cyano, C₁-C₄-alkoxy, C₁-C₄-haloalkoxy, amino, C₁-C₄-alkylamino, di(C₁-C₄)-alkylamino or C₁-C₄-thioalkyl,
X is oxygen or sulphur,
R² is a radical from the group of hydrogen, C₁-C₄-alkyl, C₁-C₄-haloalkyl, C₁-C₄-alkoxy, C₂-C₃-alkenyl, C₁-C₂-alkoxy-C₁-C₂-alkyl, C₁-C₂-alkylcarbonyl, C₁-C₂-alkoxycarbonyl and in each case optionally halogen-substituted cyclopropylcarbonyl, cyclopentylcarbonyl or cyclohexylcarbonyl,
R³ is a radical from the group of hydrogen, C₁-C₄-alkyl, C₁-C₅-haloalkyl, cyano-C₁-C₄-alkyl, C₂-C₄-alkenyl, C₂-C₄-haloalkenyl, C₂-C₄-alkynyl, C₂-C₄-haloalkynyl, C₁-C₄-alkoxy, C₁-C₄-haloalkoxy, optionally halogen-substituted C₁-C₂-alkoxy-C₁-C₄-alkyl, optionally halogen-substituted bis(C₁-C₂-alkoxy)-C₁-C₄-alkyl, optionally halogen-substituted C₁-C₄-alkylthio-C₁-C₄-alkyl, optionally halogen-substituted C₁-C₄-alkylcarbonyl-C₁-C₄-alkyl, optionally halogen-substituted C₁-C₄-alkylsulphinyl-C₁-C₄-alkyl, optionally halogen-substituted C₁-C₄-alkylsulphonyl-C₁-C₄-alkyl, optionally halogen-substituted C₁-C₄-alkoxycarbonyl-C₁-C₄-alkyl, C₁-C₄-alkoxycarbonylamino optionally C₁-C₄-alkyl-substituted on the nitrogen, C₂-C₄-alkynyloxy, C₂-C₄-alkynyloxycarbonyl, optionally halogen-, cyano-, C₁-C₄-alkyl-, C₁-C₄-haloalkyl-, C₁-C₄-alkoxy-, C₁-C₄-haloalkoxy-, C₁-C₄-alkoxycarbonyl-, C₁-C₄-haloalkoxycarbonyl- or pyridyl-substituted (where pyridyl is itself optionally substituted by C₁-C₄-alkyl or halogen) C₃-C₆-cycloalkyl, optionally halogen-, cyano-, C₁-C₄-alkyl-, C₁-C₄-haloalkyl-, C₁-C₄-alkoxy-, C₁-C₄-haloalkoxy-, C₁-C₄-alkoxycarbonyl-, C₁-C₄-haloalkoxycarbonyl- or pyridyl-substituted (where pyridyl is itself optionally substituted by C₁-C₄-alkyl or halogen) C₃-C₆-cycloalkylcarbonyl, optionally halogen-, cyano-, C₁-C₄-alkyl-, C₁-C₄-haloalkyl-, C₁-C₄-alkoxy-, C₁-C₄-haloalkoxy-, C₁-C₄-alkoxycarbonyl-, C₁-C₄-haloalkoxycarbonyl- or pyridyl-substituted (where pyridyl is itself optionally substituted by C₁-C₄-alkyl or halogen) C₃-C₆-cycloalkyl-C₁-C₄-alkyl, optionally halogen-, cyano- (including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-alkylsulphonyl-, C₁-C₄-haloalkylsulphinyl-, C₁-C₄-haloalkylsulphonyl-, amino-, C₁-C₄-alkylamino-, di (C₁-C₄-alkyl)amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted and optionally benzofused heterocyclyl-C₁-C₄-alkyl, optionally halogen-, cyano-, C₁-C₄-alkyl-, C₁-C₄-haloalkyl-, C₁-C₄-alkoxy- or C₁-C₄-haloalkoxy-substituted aryl-C₁-C₄-alkyl, aryloxy-C₁-C₄-alkyl optionally halogen-substituted in the aryl moiety and optionally halogen-, cyano- (including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkyl-sulphinyl-, C₁-C₄-alkylsulphonyl-, C₁-C₄-haloalkylsulphinyl-, C₁-C₄-haloalkylsulphonyl-, amino-, C₁-C₄-alkylamino-, di (C₁-C₄-alkyl)amino-, C₁-C₄-alkylcarbonylamino-, C₁-C₄-alkoxycarbonyl-amino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted hetaryl-C₁-C₄-alkyl
or
R² and R³ together with the nitrogen atom to which they are bonded form an optionally C₁-C₄-alkyl- or C₁-C₄-alkoxy-substituted 3- to 7-membered ring which optionally contains one or two further heteroatoms from the group of oxygen, nitrogen and sulphur, and in which
halogen (or halo) is selected from the group of fluorine, chlorine, bromine and iodine,
aryl (including as part of a larger unit, for example arylalkyl) is selected from the group of phenyl, naphthyl, anthryl, phenanthrenyl,
hetaryl (including as part of a larger unit, for example hetarylalkyl) is selected from the group of pyrimidyl, oxadiazolyl, oxazolyl, pyrazinyl, imidazolyl, thiazolyl and furanyl and
heterocyclyl (including as part of a larger unit, for example heterocyclylalkyl) is selected from the group of 2-tetrahydrofuranyl, 3-tetrahydrofuranyl, 2,3-dihydro-1-benzofur-2-yl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,3-dihydrothien-2-yl, 2,3-dihydrothien-3-yl, 2,4-dihydrothien-2-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 1,4-dioxan-2-yl, 1,3-dioxolan-2-yl, 1,3-dioxolan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl.

3. Compounds of the formula (I) according to Claim 1, in which
G is N, CH, C-halogen, C-cyano, C-CH₃, C-CF₃, C-cyclopropyl, C-OCH₃, C-OCF₃,
R¹ is hydrogen, methyl, trifluoromethyl, cyclopropyl, halogen, cyano, methoxy, trifluoromethoxy, amino, methylamino, dimethylamino,
X is oxygen or sulphur,
R² is a radical from the group of hydrogen, methyl, ethyl, CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, methoxy, ethoxy, vinyl, CH₂OCH₃, CH₂OCH₂CH₃, methylcarbonyl, ethylcarbonyl, methoxycarbonyl, ethoxycarbonyl, cyclopropylcarbonyl and fluorocyclopropylcarbonyl,
R³ is a radical from the group of hydrogen, methyl, ethyl, CH₂CF₃, CH₂CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, CH(CN)CH(CH₃)₂, CH₂CN, CH₂CH₂CN, vinyl, C(CH₃)₂CCH, CH₂CCCH₃, methoxy, ethoxy, CH₂CH(OCH₃)2, CH₂CH(CH₃)(OCH₃), CH₂C(CH₃)₂(OCH₃), CH(CH₃)CH(OCH₃)₂, CH₂C(CH₃)(OCH₃)₂, C(CH₃)₂CH₂SCH₃, CH₂CH₂SCH₃, CHCH₃CH₂SCH₃, optionally halogen-substituted C₁-C₄-alkylcarbonyl-C₁-C₄-alkyl, CH₃SO₂CH₂C(CH₃)₂, CH₃SO₂CH₂CHCH₃, propargyloxy, cyclopropyl, cyanocyclopropyl, fluorocyclopropyl, trifluoromethylcyclopropyl, trifluoromethylcyclohexyl, methoxycarbonylcyclopropyl, fluorocyclopropylcarbonyl, cyclopropylmethyl, 1-cyclopropylethyl, cyclohexylmethyl, 1-cyano-1-cyclopropyleth-1-yl, 1,3-dioxolan-2-ylmethyl, 4-methyl-1,3-dioxolan-2-ylmethyl, tetrahydrofurylmethyl, tetrahydropyranylmethyl, 2,2-dimethyl-1,3-dioxolan-5-ylmethyl, 2-methyltetrahydrofur-2-ylmethyl, α-methyl-3,5-dimethyltriazol-1-yl-ethyl, 1,5-dimethyl-1,3-oxazol-4-ylmethyl, optionally halogen-, cyano-, methyl-, ethyl-, methoxy- or ethoxy-substituted benzyl or pyrimidylmethyl, pyridylmethyl, α-methyl-pyrimidylmethyl, oxadiazolylmethyl, oxazolylmethyl, 5-methylpyrazin-2-yl, α-methylpyrid-2-ylmethyl, 4-bromopyrimid-2-ylmethyl, imidazolylmethyl, 2-methylpyrimid-4-ylmethyl, 6-chloropyridin-3-ylmethyl, 4,6-dimethylpyrimid-2-ylmethyl, thiazolylmethyl, furanylmethyl, 1,5-dimethylpyrazol-3-ylmethyl, 4-iodopyrimid-2-ylmethyl, 3-cyclopropyl-1,2,4-oxadiazol-5-ylmethyl, 2-ethylpyrimid-6-ylmethyl, 6-bromopyrid-2-ylmethyl, (2,3-dihydro-1-benzofur-2-yl)methyl, (1,4-dioxan-2-yl)methyl, 1,3-dioxolan-2-ylmethyl, methoxycarbonylethyl (methylalaninate), methoxycarbonylamino, 4,6-dimethoxypyrimid-2-ylmethyl, 2-(2,5-dichlorophenoxy)ethyl and N-methyl-N-methoxycarbonylamino,
or
R² and R³ together with the nitrogen atom to which they are bonded form an optionally C₁-C₄-alkyl- or C₁-C₄-alkoxy-substituted 3- to 7-membered ring which optionally contains one or two further heteroatoms from the group of oxygen, nitrogen and sulphur.

4. Process for preparing compounds of the formula (I) according to Claim 1, **characterized in that**
compounds of the formula (II) 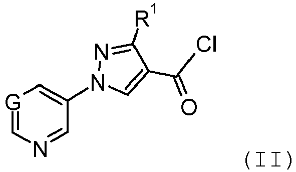 in which
G and R¹ are each as defined in Claim 1
are reacted with compounds of the formula (III) 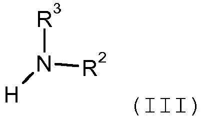 in which
R² and R³ are each as defined in Claim 1,
optionally in the presence of a suitable diluent and optionally in the presence of a base, and the compounds of the formula (I) in which X is oxygen, obtainable by this process, are optionally converted by reaction with a thionating agent to compounds of the formula (I) in which X is sulphur.

5. Composition, **characterized by** a content of at least one compound of the formula (I) according to Claims 1 to 3 and customary extenders and/or surfactants.

6. Method for controlling pests, **characterized in that** a compound of the formula (I) according to Claims 1 to 3 or a composition according to Claim 5 is allowed to act on the pests and/or their habitat.

7. Use of compounds of the formula (I) according to Claims 1 to 3 or of compositions according to Claim 5 for controlling pests.

## Revendications

1. Composés de formule (I) 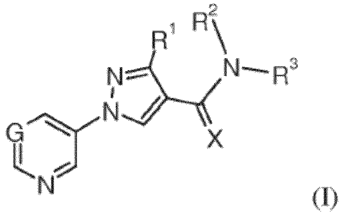 dans lesquels
G représente N, CH, C-halogène, C-nitro, C-cyano, C-alkyle en C₁-C₆, C-haloalkyle en C₁-C₆, C-cycloalkyle en C₃-C₆, C-alcoxy en C₁-C₆, C-haloalcoxy en C₁-C₆,
R¹ représente hydrogène, alkyle en C₁-C₆, haloalkyle en C₁-C₆, cycloalkyle en C₃-C₆, halogène, cyano, alcoxy en C₁-C₆, haloalcoxy en C₁-C₆, amino, alkylamino en C₁-C₆, di-alkylamino en C₁-C₆ ou thioalkyle en C₁-C₆,
X représente oxygène ou soufre,
R² représente un radical de la série constituée par hydrogène, alkyle en C₁-C₆, halogénoalkyle en C₁-C₆, alcoxy en C₁-C₄, halogénoalcoxy en C₁-C₄, alcényle en C₂-C₄, alcoxy en C₁-C₄-alkyle en C₁-C₄, alkylcarbonyle en C₁-C₄ éventuellement substitué par halogène, alcoxycarbonyle en C₁-C₄ éventuellement substitué par halogène et cycloalkylcarbonyle en C₃-C₆ éventuellement substitué par halogène,
R³ représente un radical de la série constituée par hydrogène, alkyle en C₁-C₆, halogénoalkyle en C₁-C₆, cyanoalkyle en C₁-C₆, alcényle en C₂-C₄, halogénoalcényle en C₂-C₄, alcynyle en C₂-C₄, halogénoalcynyle en C₂-C₄, alcoxy en C₁-C₆, halogénoalcoxy en C₁-C₆, alcoxy en C₁-C₆-alkyle en C₁-C₆ éventuellement substitué par halogène, bis (alcoxy en C₁-C₆) alkyle en C₁-C₆ éventuellement substitué par halogène, alkylthio en C₁-C₆-alkyle en C₁-C₆ éventuellement substitué par halogène, alkylcarbonyle en C₁-C₄-alkyle en C₁-C₄ éventuellement substitué par halogène, alkylsulfinyle en C₁-C₆-alkyle en C₁-C₆ éventuellement substitué par halogène, alkylsulfonyle en C₁-C₆-alkyle en C₁-C₆ éventuellement substitué par halogène, alcoxycarbonyle en C₁-C₆-alkyle en C₁-C₆ éventuellement substitué par halogène, alcoxycarbonylamino en C₁-C₆ éventuellement substitué par alkyle en C₁-C₆ sur l'azote, alcynyloxy en C₂-C₄, alcynyloxycarbonyle en C₂-C₄, cycloalkyle en C₃-C₆ éventuellement substitué par halogène, cyano, alkyle en C₁-C₆, halogénoalkyle en C₁-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est éventuellement lui-même substitué par alkyle en C₁-C₆ ou halogène), cycloalkylcarbonyle en C₃-C₆ éventuellement substitué par halogène, cyano, alkyle en C₁-C₆, halogénoalkyle en C₁-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est éventuellement lui-même substitué par alkyle en C₁-C₆ ou halogène), cycloalkyle en C₃-C₆-alkyle en C₁-C₆ éventuellement substitué par halogène, cyano, alkyle en C₁-C₆, halogénoalkyle en C₁-C₆, alcoxy en C₁-C₆, halogénoalcoxy en C₁-C₆, alcoxycarbonyle en C₁-C₆, halogénoalcoxycarbonyle en C₁-C₆ ou hétaryle (qui est éventuellement lui-même substitué par alkyle en C₁-C₆ ou halogène), hétérocyclyl-alkyle en C₁-C₆ éventuellement substitué par halogène, cyano (également dans la partie alkyle), nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, haloalkylthio en C₁-C₆, alkylsulfinyle en C₁-C₆, alkylsulfonyle en C₁-C₆, haloalkylsulfinyle en C₁-C₆, haloalkylsulfonyle en C₁-C₆, amino, alkylamino en C₁-C₆, di (alkyle en C₁-C₆) amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle, et éventuellement benzocondensé, aryl-alkyle en C₁-C₆ éventuellement substitué par halogène, cyano, alkyle en C₁-C₆, halogénoalkyle en C₁-C₆, alcoxy en C₁-C₆ ou halogénoalcoxy en C₁-C₆, aryloxy-alkyle en C₁-C₆ éventuellement substitué dans la partie aryle par halogène, hétaryl-alkyle en C₁-C₆ éventuellement substitué par halogène, cyano (également dans la partie alkyle), nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, haloalkylthio en C₁-C₆, alkylsulfinyle en C₁-C₆, alkylsulfonyle en C₁-C₆, haloalkylsulfinyle en C₁-C₆, haloalkylsulfonyle en C₁-C₆, amino, alkylamino en C₁-C₆, di (alkyle en C₁-C₆) amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle, et NR⁴R⁵, R⁴ et R⁵ représentant indépendamment l'un de l'autre un radical de la série constituée par hydrogène, alkyle en C₁-C₆, halogénoalkyle en C₁-C₆, cycloalkyle en C₃-C₆, alcoxy en C₁-C₆, alkylcarbonyle en C₁-C₆ et alcoxycarbonyle en C₁-C₆,
ou
R² et R³ forment ensemble avec l'atome d'azote auquel ils sont reliés un cycle de 3 à 7 éléments éventuellement substitué par alkyle en C₁-C₆ ou alcoxy en C₁-C₆ et contenant éventuellement un ou deux hétéroatomes supplémentaires de la série constituée par oxygène, azote et soufre,
halogène (ou halo) étant choisi dans la série constituée par fluor, chlore, brome et iode,
aryle (également en tant que partie d'une unité plus grande, telle que par exemple arylalkyle) étant choisi dans la série constituée par phényle, naphtyle, anthryle, phénanthryle,
hétaryle (également en tant que partie d'une unité plus grande, telle que par exemple hétarylalkyle) étant choisi dans la série constituée par furyle, thiényle, pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phtalazinyle, quinazolinyle, quinoxalinyle, naphtyridinyle, benzotriazinyle, purinyle, ptéridinyle et indolizinyle et
hétérocyclyle (également en tant que partie d'une unité plus grande, telle que par exemple hétérocyclylalkyle) étant choisi dans la série constituée par 2-tétrahydrofuranyle, 3-tétrahydrofuranyle, 2,3-dihydro-1-benzofur-2-yle, 2-tétrahydrothiényle, 3-tétrahydrothiényle, 2-pyrrolidinyle, 3-pyrrolidinyle, 3-isoxazolidinyle, 4-isoxazolidinyle, 5-isoxazolidinyle, 3-isothiazolidinyle, 4-isothiazolidinyle, 5-isothiazolidinyle, 3-pyrazolidinyle, 4-pyrazolidinyle, 5-pyrazolidinyle, 2-oxazolidinyle, 4-oxazolidinyle, 5-oxazolidinyle, 2-thiazolidinyle, 4-thiazolidinyle, 5-thiazolidinyle, 2-imidazolidinyle, 4-imidazolidinyle, 1,2,4-oxadiazolidin-3-yle, 1,2,4-oxadiazolidin-5-yle, 1,2,4-thiadiazolidin-3-yle, 1,2,4-thiadiazolidin-5-yle, 1,2,4-triazolidin-3-yle, 1,3,4-oxadiazolidin-2-yle, 1,3,4-thiadiazolidin-2-yle, 1,3,4-triazolidin-2-yle, 2,3-dihydrofur-2-yle, 2,3-dihydrofur-3-yle, 2,4-dihydrofur-2-yle, 2,3-dihydrothién-2-yle, 2,3-dihydrothién-3-yle, 2,4-dihydrothién-2-yle, 2-pyrrolin-2-yle, 2-pyrrolin-3-yle, 3-pyrrolin-2-yle, 3-pyrrolin-3-yle, 2-isoxazolin-3-yle, 3-isoxazolin-3-yle, 4-isoxazolin-3-yle, 2-isoxazolin-4-yle, 3-isoxazolin-4-yle, 4-isoxazolin-4-yle, 2-isoxazolin-5-yle, 3-isoxazolin-5-yle, 4-isoxazolin-5-yle, 2-isothiazolin-3-yle, 3-isothiazolin-3-yle, 4-isothiazolin-3-yle, 2-isothiazolin-4-yle, 3-isothiazolin-4-yle, 4-isothiazolin-4-yle, 2-isothiazolin-5-yle, 3-isothiazolin-5-yle, 4-isothiazolin-5-yle, 2,3-dihydropyrazol-1-yle, 2,3-dihydropyrazol-2-yle, 2,3-dihydropyrazol-3-yle, 2,3-dihydropyrazol-4-yle, 2,3-dihydropyrazol-5-yle, 3,4-dihydropyrazol-1-yle, 3,4-dihydropyrazol-3-yle, 3,4-dihydropyrazol-4-yle, 3,4-dihydropyrazol-5-yle, 4,5-dihydropyrazol-1-yle, 4,5-dihydropyrazol-3-yle, 4,5-dihydropyrazol-4-yle, 4,5-dihydropyrazol-5-yle, 2,3-dihydrooxazol-2-yle, 2,3-dihydrooxazol-3-yle, 2,3-dihydrooxazol-4-yle, 2,3-dihydrooxazol-5-yle, 3,4-dihydrooxazol-2-yle, 3,4-dihydrooxazol-3-yle, 3,4-dihydrooxazol-4-yle, 3,4-dihydrooxazol-5-yle, 3,4-dihydrooxazol-2-yle, 3,4-dihydrooxazol-3-yle, 3,4-dihydrooxazol-4-yle, 2-pipéridinyle, 3-pipéridinyle, 4-pipéridinyle, 1,3-dioxan-5-yle, 1,4-dioxan-2-yle, 1,3-dioxolan-2-yle, 1,3-dioxolan-5-yle, 2-tétrahydropyranyle, 4-tétrahydropyranyle, 2-tétrahydrothiényle, 3-hexahydropyridazinyle, 4-hexahydropyridazinyle, 2-hexahydropyrimidinyle, 4-hexahydropyrimidinyle, 5-hexahydropyrimidinyle, 2-pipérazinyle, 1,3,5-hexahydrotriazin-2-yle et 1,2,4-hexahydrotriazin-3-yle.

2. Composés de formule (I) selon la revendication 1, dans lesquels
G représente N, CH, C-halogène, C-nitro, C-cyano, C-alkyle en C₁-C₄, C-haloalkyle en C₁-C₄, C-cycloalkyle en C₃-C₆, C-alcoxy en C₁-C₄, C-haloalcoxy en C₁-C₄,
R¹ représente hydrogène, alkyle en C₁-C₄, haloalkyle en C₁-C₄, cycloalkyle en C₃-C₆, halogène, cyano, alcoxy en C₁-C₄, haloalcoxy en C₁-C₄, amino, alkylamino en C₁-C₄, di-alkylamino en C₁-C₄ ou thioalkyle en C₁-C₄,
X représente oxygène ou soufre,
R² représente un radical de la série constituée par hydrogène, alkyle en C₁-C₄, halogénoalkyle en C₁-C₄, alcoxy en C₁-C₄, alcényle en C₂-C₃, alcoxy en C₁-C₂-alkyle en C₁-C₂, alkylcarbonyle en C₁-C₂, alcoxycarbonyle en C₁-C₂; et cyclopropylcarbonyle, cyclopentylcarbonyle ou cyclohexylcarbonyle, chacun éventuellement substitué par halogène,
R³ représente un radical de la série constituée par hydrogène, alkyle en C₁-C₄, halogénoalkyle en C₁-C₅, cyanoalkyle en C₁-C₄, alcényle en C₂-C₄, halogénoalcényle en C₂-C₄, alcynyle en C₂-C₄, halogénoalcynyle en C₂-C₄, alcoxy en C₁-C₄, halogénoalcoxy en C₁-C₄, alcoxy en C₁-C₂-alkyle en C₁-C₄ éventuellement substitué par halogène, bis (alcoxy en C₁-C₂) alkyle en C₁-C₄ éventuellement substitué par halogène, alkylthio en C₁-C₄-alkyle en C₁-C₄ éventuellement substitué par halogène, alkylcarbonyle en C₁-C₄-alkyle en C₁-C₄ éventuellement substitué par halogène, alkylsulfinyle en C₁-C₄-alkyle en C₁-C₄ éventuellement substitué par halogène, alkylsulfonyle en C₁-C₄-alkyle en C₁-C₄ éventuellement substitué par halogène, alcoxycarbonyle en C₁-C₄-alkyle en C₁-C₄ éventuellement substitué par halogène, alcoxycarbonylamino en C₁-C₄ éventuellement substitué par alkyle en C₁-C₄ sur l'azote, alcynyloxy en C₂-C₄, alcynyloxycarbonyle en C₂-C₄, cycloalkyle en C₃-C₆ éventuellement substitué par halogène, cyano, alkyle en C₁-C₄, halogénoalkyle en C₁-C₄, alcoxy en C₁-C₄, halogénoalcoxy en C₁-C₄, alcoxycarbonyle en C₁-C₄, halogénoalcoxycarbonyle en C₁-C₄ ou pyridyle (qui est éventuellement lui-même substitué par alkyle en C₁-C₄ ou halogène), cycloalkylcarbonyle en C₃-C₆ éventuellement substitué par halogène, cyano, alkyle en C₁-C₄, halogénoalkyle en C₁-C₄, alcoxy en C₁-C₄, halogénoalcoxy en C₁-C₄, alcoxycarbonyle en C₁-C₄, halogénoalcoxycarbonyle en C₁-C₄ ou pyridyle (qui est éventuellement lui-même substitué par alkyle en C₁-C₄ ou halogène), cycloalkyle en C₃-C₆-alkyle en C₁-C₄ éventuellement substitué par halogène, cyano, alkyle en C₁-C₄, halogénoalkyle en C₁-C₄, alcoxy en C₁-C₄, halogénoalcoxy en C₁-C₄, alcoxycarbonyle en C₁-C₄, halogénoalcoxycarbonyle en C₁-C₄ ou pyridyle (qui est éventuellement lui-même substitué par alkyle en C₁-C₄ ou halogène), hétérocyclyl-alkyle en C₁-C₄ éventuellement substitué par halogène, cyano (également dans la partie alkyle), nitro, alkyle en C₁-C₄, halogénoalkyle en C₁-C₄, cycloalkyle en C₃-C₆, alcoxy en C₁-C₄, halogénoalcoxy en C₁-C₄, alkylthio en C₁-C₄, haloalkylthio en C₁-C₄, alkylsulfinyle en C₁-C₄, alkylsulfonyle en C₁-C₄, haloalkylsulfinyle en C₁-C₄, haloalkylsulfonyle en C₁-C₄, amino, alkylamino en C₁-C₄, di (alkyle en C₁-C₄) amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle, et éventuellement benzocondensé, aryl-alkyle en C₁-C₄ éventuellement substitué par halogène, cyano, alkyle en C₁-C₄, halogénoalkyle en C₁-C₄, alcoxy en C₁-C₄ ou halogénoalcoxy en C₁-C₄, aryloxy-alkyle en C₁-C₄ éventuellement substitué dans la partie aryle par halogène, et hétaryl-alkyle en C₁-C₄ éventuellement substitué par halogène, cyano (également dans la partie alkyle), nitro, alkyle en C₁-C₄, halogénoalkyle en C₁-C₄, cycloalkyle en C₃-C₆, alcoxy en C₁-C₄, halogénoalcoxy en C₁-C₄, alkylthio en C₁-C₄, haloalkylthio en C₁-C₄, alkylsulfinyle en C₁-C₄, alkylsulfonyle en C₁-C₄, haloalkylsulfinyle en C₁-C₄, haloalkylsulfonyle en C₁-C₄, amino, alkylamino en C₁-C₄, di(alkyle en C₁-C₄) amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle,
ou
R² et R³ forment ensemble avec l'atome d'azote auquel ils sont reliés un cycle de 3 à 7 éléments éventuellement substitué par alkyle en C₁-C₄ ou alcoxy en C₁-C₄ et contenant éventuellement un ou deux hétéroatomes supplémentaires de la série constituée par oxygène, azote et soufre,
halogène (ou halo) étant choisi dans la série constituée par fluor, chlore, brome et iode,
aryle (également en tant que partie d'une unité plus grande, telle que par exemple arylalkyle) étant choisi dans la série constituée par phényle, naphtyle, anthryle, phénanthryle,
hétaryle (également en tant que partie d'une unité plus grande, telle que par exemple hétarylalkyle) étant choisi dans la série constituée par pyrimidyle, oxadiazolyle, oxazolyle, pyrazinyle, imidazolyle, thiazolyle et furanyle, et
hétérocyclyle (également en tant que partie d'une unité plus grande, telle que par exemple hétérocyclylalkyle) étant choisi dans la série constituée par 2-tétrahydrofuranyle, 3-tétrahydrofuranyle, 2,3-dihydro-1-benzofur-2-yle, 2-tétrahydrothiényle, 3-tétrahydrothiényle, 2-pyrrolidinyle, 3-pyrrolidinyle, 3-isoxazolidinyle, 4-isoxazolidinyle, 5-isoxazolidinyle, 3-isothiazolidinyle, 4-isothiazolidinyle, 5-isothiazolidinyle, 3-pyrazolidinyle, 4-pyrazolidinyle, 5-pyrazolidinyle, 2-oxazolidinyle, 4-oxazolidinyle, 5-oxazolidinyle, 2-thiazolidinyle, 4-thiazolidinyle, 5-thiazolidinyle, 2-imidazolidinyle, 4-imidazolidinyle, 1,2,4-oxadiazolidin-3-yle, 1,2,4-oxadiazolidin-5-yle, 1,2,4-thiadiazolidin-3-yle, 1,2,4-thiadiazolidin-5-yle, 1,2,4-triazolidin-3-yle, 1,3,4-oxadiazolidin-2-yle, 1,3,4-thiadiazolidin-2-yle, 1,3,4-triazolidin-2-yle, 2,3-dihydrofur-2-yle, 2,3-dihydrofur-3-yle, 2,4-dihydrofur-2-yle, 2,3-dihydrothién-2-yle, 2,3-dihydrothién-3-yle, 2,4-dihydrothién-2-yle, 2-pyrrolin-2-yle, 2-pyrrolin-3-yle, 3-pyrrolin-2-yle, 3-pyrrolin-3-yle, 2-isoxazolin-3-yle, 3-isoxazolin-3-yle, 4-isoxazolin-3-yle, 2-isoxazolin-4-yle, 3-isoxazolin-4-yle, 4-isoxazolin-4-yle, 2-isoxazolin-5-yle, 3-isoxazolin-5-yle, 4-isoxazolin-5-yle, 2-isothiazolin-3-yle, 3-isothiazolin-3-yle, 4-isothiazolin-3-yle, 2-isothiazolin-4-yle, 3-isothiazolin-4-yle, 4-isothiazolin-4-yle, 2-isothiazolin-5-yle, 3-isothiazolin-5-yle, 4-isothiazolin-5-yle, 2,3-dihydropyrazol-1-yle, 2,3-dihydropyrazol-2-yle, 2,3-dihydropyrazol-3-yle, 2,3-dihydropyrazol-4-yle, 2,3-dihydropyrazol-5-yle, 3,4-dihydropyrazol-1-yle, 3,4-dihydropyrazol-3-yle, 3,4-dihydropyrazol-4-yle, 3,4-dihydropyrazol-5-yle, 4,5-dihydropyrazol-1-yle, 4,5-dihydropyrazol-3-yle, 4,5-dihydropyrazol-4-yle, 4,5-dihydropyrazol-5-yle, 2,3-dihydrooxazol-2-yle, 2,3-dihydrooxazol-3-yle, 2,3-dihydrooxazol-4-yle, 2,3-dihydrooxazol-5-yle, 3,4-dihydrooxazol-2-yle, 3,4-dihydrooxazol-3-yle, 3,4-dihydrooxazol-4-yle, 3,4-dihydrooxazol-5-yle, 3,4-dihydrooxazol-2-yle, 3,4-dihydrooxazol-3-yle, 3,4-dihydrooxazol-4-yle, 2-pipéridinyle, 3-pipéridinyle, 4-pipéridinyle, 1,3-dioxan-5-yle, 1,4-dioxan-2-yle, 1,3-dioxolan-2-yle, 1,3-dioxolan-5-yle, 2-tétrahydropyranyle, 4-tétrahydropyranyle, 2-tétrahydrothiényle, 3-hexahydropyridazinyle, 4-hexahydropyridazinyle, 2-hexahydropyrimidinyle, 4-hexahydropyrimidinyle, 5-hexahydropyrimidinyle, 2-pipérazinyle, 1,3,5-hexahydrotriazin-2-yle et 1,2,4-hexahydrotriazin-3-yle.

3. Composés de formule (I) selon la revendication 1, dans lesquels
G représente N, CH, C-halogène, C-cyano, C-CH₃, C-CF₃, C-cyclopropyle, C-OCH₃, C-OCF₃,
R¹ représente hydrogène, méthyle, trifluorométhyle, cyclopropyle, halogène, cyano, méthoxy, trifluorométhoxy, amino, méthylamino, diméthylamino,
X représente oxygène ou soufre,
R² représente un radical de la série constituée par hydrogène, méthyle, éthyle, CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, méthoxy, éthoxy, vinyle, CH₂OCH₃, CH₂OCH₂CH₃, méthylcarbonyle, éthylcarbonyle, méthoxycarbonyle, éthoxycarbonyle, cyclopropylcarbonyle et fluorocyclopropylcarbonyle,
R³ représente un radical de la série constituée par hydrogène, méthyle, éthyle, CH₂CF₃, CH₂CH₂CF₃, CH₂CF₂CH₃, CH₂CHF₂, CH₂CF₂CHF₂, CH₂CH₂F, CH₂CHFCH₃, CH₂CF₂Br, CH₂CFCl₂, CH(CH₃)CH₂F, CH₂CCl₃, CH₂CClF₂, CH₂CH₂CH₂F, CH₂CH(CH₃)Cl, CHCF₃CH(CH₃)₂, CH(CF₃)₂, CH₂CH₂Cl, CHCF₃CH₂CH₂CH₃, CH₂CF₂CF₃, C(CH₃)₂CN, CH(CN)CH(CH₃)₂, CH₂CN, CH₂CH₂CN, vinyle, C(CH₃)₂CCH, CH₂CCCH₃, méthoxy, éthoxy, CH₂CH(OCH₃)₂, CH₂CH(CH₃) (OCH₃), CH₂C(CH₃)₂(OCH₃), CH(CH₃)CH(OCH₃)₂, CH₂C(CH₃)(OCH₃)₂, C(CH₃)₂CH₂SCH₃, CH₂CH₂SCH₃, CHCH₃CH₂SCH₃, alkylcarbonyle en C₁-C₄-alkyle en C₁-C₄ éventuellement substitué par halogène, CH₃SO₂CH₂C(CH₃)₂, CH₃SO₂CH₂CHCH₃, propargyloxy, cyclopropyle, cyanocyclopropyle, fluorocyclopropyle, trifluorométhylcyclopropyle, trifluorométhylcyclohexyle, méthoxycarbonylcyclopropyle, fluorocyclopropylcarbonyle, cyclopropylméthyle, 1-cyclopropyléthyle, cyclohexylméthyle, 1-cyano-1-cyclopropyl-éth-1-yle, 1,3-dioxolan-2-ylméthyle, 4-méthyl-1,3-dioxolan-2-ylméthyle, tétrahydrofurylméthyle, tétrahydropyranylméthyle, 2,2-diméthyl-1,3-dioxolan-5-yl-méthyle, 2-méthyltétrahydrofur-2-yl-méthyle, α-méthyl-3,5-diméthyltriazol-1-yl-éthyle, 1,5-diméthyl-1,3-oxazol-4-yl-méthyle, benzyle éventuellement substitué par halogène, cyano, méthyle, éthyle, méthoxy ou éthoxy ; pyrimidylméthyle, pyridylméthyle, α-méthyl-pyrimidylméthyle, oxadiazolylméthyle, oxazolylméthyle, 5-méthyl-pyrazin-2-yle, α-méthyl-pyrid-2-yl-méthyle, 4-bromo-pyrimid-2-yl-méthyle, imidazolylméthyle, 2-méthylpyrimid-4-yl-méthyle, 6-chloro-pyridin-3-yl-méthyle, 4,6-diméthylpyrimid-2-yl-méthyle, thiazolylméthyle, furanylméthyle, 1,5-diméthylpyrazol-3-yl-méthyle, 4-iodo-pyrimid-2-yl-méthyle, 3-cyclopropyl-1,2,4-oxdiazol-5-yl-méthyle, 2-éthyl-pyrimid-6-yl-méthyle, 6-bromo-pyrid-2-yl-méthyle, (2,3-dihydro-1-benzofur-2-yl)méthyle, (1,4-dioxan-2-yl)méthyle, 1,3-dioxolan-2-ylméthyle, méthoxycarbonyléthyle (alaninate de méthyle), méthoxycarbonylamino, 4,6-diméthoxypyrimid-2-ylméthyle, 2-(2,5-dichlorophénoxy)éthyle et N-méthyl-N-méthoxycarbonyl-amino,
ou
R² et R³ forment ensemble avec l'atome d'azote auquel ils sont reliés un cycle de 3 à 7 éléments éventuellement substitué par alkyle en C₁-C₄ ou alcoxy en C₁-C₄ et contenant éventuellement un ou deux hétéroatomes supplémentaires de la série constituée par oxygène, azote et soufre.

4. Procédé de fabrication de composés de formule (I) selon la revendication 1, **caractérisé en ce que** des composés de formule (II) 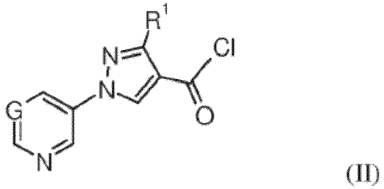 dans laquelle
G et R¹ ont les significations indiquées dans la revendication 1,
sont mis en réaction avec des composés de formule (III) 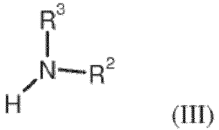 dans laquelle
R² et R³ ont les significations indiquées dans la revendication 1,
éventuellement en présence d'un diluant approprié et éventuellement en présence d'une base, et les composés de formule (I) pouvant être obtenus par ce procédé, dans lesquels X représente oxygène, sont éventuellement transformés en des composés de formule (I) dans lesquels X représente soufre par réaction avec un réactif de sulfuration.

5. Agent, **caractérisé par** une teneur en au moins un composé de formule (I) selon les revendications 1 à 3 et des diluants et/ou tensioactifs usuels.

6. Procédé de lutte contre des organismes nuisibles, **caractérisé en ce qu'**un composé de formule (I) selon les revendications 1 à 3 ou un agent selon la revendication 5 est laissé agir sur les organismes nuisibles et/ou leur habitat.

7. Utilisation de composés de formule (I) selon les revendications 1 à 3 ou d'agents selon la revendication 5 pour lutter contre des organismes nuisibles.
